# EUROPEAN PATENT SPECIFICATION

(11) **EP 0 778 277 B1**
(45) Date of publication and mention of the grant of the patent: **25.06.2003**
(21) Application number: 96308092.4
(22) Date of filing: 07.11.1996
(51) Int. Cl.: C07D 487/04, A61K 31/52, C07D 471/04, C07D 473/34, C07D 498/04

(54) **Substituted heterocyclic derivatives as CRF antagonists**
Substitutierte heterozyclische Derivate als CRF Antagonisten
Derivés hétérocycliques substitués comme CRF antagonistes

(30) Priority: 08.12.1995 US 8396
(43) Date of publication of application: 11.06.1997
(73) Proprietor: PFIZER INC., New York, N.Y. 10017 (US)
(72) Inventor: Chen, Yuhpyng L., Waterford, Connecticut 06385 (US)
(74) Representative: Eddowes, Simon

(56) References cited:
- EP-A- 0 157 637
- WO-A-94/13676
- WO-A-94/13677
- US-A- 4 725 601
- US-A- 4 904 666
- US-A- 5 028 605

## Description

### Background of the Invention

This invention relates to certain pharmaceutically active substituted heterocyclic derivatives, pharmaceutical compositions containing them and methods of administering them to subjects in need of their corticotropin releasing factor antagonist activity.

The substituted heterocyclic derivatives claimed in this case exhibit activity as corticotropin releasing factor CRF antagonists.

CRF antagonists are referred to in U.S. Patents 4,605,642 and 5,063,245, which relate, respectively, to peptides and pyrazolinones, and were issued, respectively, on August 12, 1986 and November 5, 1991. They are also referred to in the following: PCT Patent Application PCT/IB95/00439, which designates the United States and was filed on June 6, 1995; PCT Patent Application PCT/IB95/00373, which designates the United States and was filed on May 18, 1995; U.S. Patent Application 08/448,539, which was filed in the PCT on Nov. 12, 1993 and entered the U.S. national phase on June 14, 1995; U.S. Patent Application 08/481,413, which was filed in the PCT on November 26, 1993 and entered the U.S. national phase on July 24, 1995; and U.S. Patent Application 08/254,820, which was filed on April 19, 1995. All the foregoing patents and patent applications are incorporated herein by reference in their entireties.

The importance of CRF antagonists is discussed in the literature, e.g., as discussed in U.S. Patent 5,063,245, which is incorporated herein by reference in its entirety. A recent outline of the different activities possessed by CRF antagonists is found in M. J. Owens et al., Pharm. Rev., Vol. 43, pages 425 to 473 (1991), also incorporated herein by reference. Based on the research described in these two and other references, CRF antagonists are effective in the treatment of a wide range of stress-related illnesses, such as depression, anxiety, headache, irritable bowel syndrome, inflammatory diseases, immune suppression, Alzheimer's disease, gastrointestinal diseases, anorexia nervosa, hemorrhagic stress, drug and alcohol withdrawal symptoms, drug addiction, infertility, head trauma, stroke, and stress-induced infections in humans and animals.

### Summary of the Invention

The present invention relates to compounds of the formula 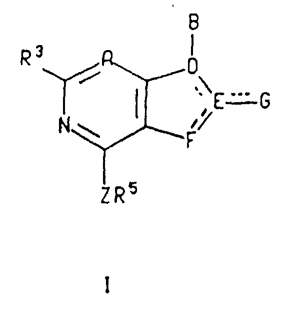 or a pharmaceutically acceptable salt thereof, wherein
the dashed lines represent optional double bonds;
A is nitrogen or CR⁷;
B is -NR¹R², -CR¹R²R¹⁰, -C(=CR²R¹¹)R¹, -NHCR¹R²R¹⁰, -OCR¹R²R¹⁰, -SCR¹R²R¹⁰, -CR²R¹⁰NHR¹, -CR²R¹⁰OR¹, -CR²R¹⁰SR¹ or -COR²;
D is nitrogen and is single bonded to all atoms to which it is attached;
E is nitrogen, CH or carbon;
F is oxygen, sulfur, CHR⁴ or NR⁴ when it is single bonded to E and F is nitrogen or CR⁴ when it is double bonded to E;
G, when single bonded to E, is hydrogen, C₁-C₄ alkyl, -S(C₁-C₄ alkyl), -O(C₁-C₄ alkyl), NH₂, -NH(C₁-C₄ alkyl) or -N(C₁-C₂ alkyl)(C₁-C₄ alkyl), wherein each of the C₁-C₄ alkyl groups of G may optionally be substituted with one hydroxy, -O(C₁-C₂ alkyl) or fluoro group; G, when double bonded to E, is oxygen, sulfur or NH; and G, when E is nitrogen and double bonded to D or F, is absent;
R¹ is C₁-C₆ alkyl optionally substituted with one or two substituents R⁸ independently selected from hydroxy, fluoro, chloro, bromo, iodo, C₁-C₄ alkoxy, CF₃, -C(=O)0-(C₁-C₄)alkyl, -OC(=O)(C₁-C₄ alkyl), -OC(=O)N(C₁-C₄ alkyl)(C₁-C₂ alkyl), -NHCO(C₁-C₄ alkyl), -COOH, -COO(C₁-C₄ alkyl), -CONH(C₁-C₄ alkyl), -CON(C₁-C₄ alkyl)(C₁-C₂ alkyl), -S(C₁-C₄ alkyl), -CN, -NO₂, -SO(C₁-C₄ alkyl), -SO₂(C₁-C₄ alkyl), -SO₂NH(C₁-C₄ alkyl) and -SO₂N(C₁-C₄ alkyl)(C₁-C₂ alkyl), wherein each of the C₁-C₄ alkyl groups in the foregoing R¹ groups may optionally contain one or two double or triple bonds;
R² is C₁-C₁₂ alkyl which may optionally contain from one to three double or triple bonds, aryl or (C₁-C₄ alkylene)aryl, wherein said aryl and the aryl moiety of said (C₁-C₄ alkylene)aryl is selected from phenyl, naphthyl, thienyl, benzothienyl, pyridyl, quinolyl, pyrazinyl, pyrimidinyl, imidazolyl, furanyl, benzofuranyl, benzothiazolyl, isothiazolyl, pyrazolyl, pyrrolyl, indolyl, pyrrolopyridyl, oxazolyl and benzoxazolyl; C₃-C₈ cycloalkyl or (C₁-C₆ alkylene)(C₃-C₈ cycloalkyl), wherein one or two of the carbon atoms of said cycloalkyl and the 5 to 8 membered cycloalkyl moieties of said (C₁-C₆ alkylene)(C₃-C₈ cycloalkyl) may optionally and independently be replaced by an oxygen or sulfur atom or by NZ² wherein Z² is selected from hydrogen, C₁-C₄ alkyl, benzyl and C₁-C₄ alkenoyl, and wherein each of the foregoing R² groups may optionally be substituted with from one to three substituents independently selected from chloro, fluoro, hydroxy and C₁-C₄ alkyl, or with one substituent selected from bromo, iodo, C₁-C₆ alkoxy, -OC(=O)(C₁-C₆ alkyl), -OC(=O)N(C₁-C₄ alkyl)(C₁-C₂ alkyl), -S(C₁-C₆ alkyl), amino, -NH(C₁-C₂ alkyl), -N(C₁-C₂ alkyl)(C₁-C₄ alkyl), -N(C₁-C₄ alkyl)-CO-(C₁-C₄ alkyl), -NHCO(C₁-C₄ alkyl), -COOH, -COO(C₁-C₄ alkyl), -CONH(C₁-C₄ alkyl), -CON(C₁-C₄ alkyl)(C₁-C₂ alkyl), -SH, -CN, -NO₂, -SO(C₁-C₄ alkyl), -SO₂(C₁-C₄ alkyl), -SO₂NH(C₁-C₄ alkyl) and -SO₂N(C₁-C₄ alkyl)(C₁-C₂ alkyl);
-NR¹R² may form a saturated 3 to 8 membered carbocyclic rin which may optionally contain from one to three double bonds and wherein one or two of the ring carbon atoms of such 5 to 8 membered rings may optionally and independently be replaced by an oxygen or sulfur atom or by NZ³ wherein Z³ is hydrogen, C₁-C₄ alkyl, benzyl or C₁-C₄ alkanoyl;
R³ is hydrogen, C₁-C₄ alkyl, -O(C₁-C₄ alkyl), chloro, fluoro, bromo, iodo, -CN -S(C₁-C₄ alkyl) or -SO₂(C₁-C₄ alkyl) wherein each of the (C₁-C₄ alkyl) moieties in the foregoing R³ groups may optionally be substituted with one substituent R⁹ selectee from hydroxy, fluoro and (C₁-C₂ alkoxy);
each R⁴ is, independently, hydrogen, (C₁-C₆ alkyl), fluoro, chloro, bromo, iodo hydroxy, cyano, amino, nitro, -O(C₁-C₄ alkyl), -N(C₁-C₄ alkyl)(C₁-C₂ alkyl), -S(C₁-C₄ alkyl), -SO(C₁-C₄ alkyl), -SO₂(C₁-C₄)alkyl, -CO(C₁-C₄ alkyl), -C(=O)H or -C(=O)O(C₁-C₄alkyl), wherein each of the (C₁-C₆ alkyl) and (C₁-C₄ alkyl) moieties in the foregoing R⁴ groups may optionally contain one or two double or triple bonds and may optionally be substituted with one or two substituents independently selected from hydroxy, amino, C₁-C₃ alkoxy, dimethylamino, methylamino, ethylamino, -NHC(=O)CH₃, fluoro, chloro, C₁-C₃ thioalkyl, -CN, -COOH, -C(=O)O(C₁-C₄ alkyl), -C(=O)(C₁-C₄ alkyl) and -NO₂;
R⁵ is phenyl, naphthyl, thienyl, benzothienyl, pyridyl, quinolyl, pyrazinyl, furanyl, benzofuranyl, benzothiazolyl, benzisothiazolyl, benzisoxazolyl, benzimidazolyl, indolyl, benzoxazolyl or C₃-C₈ cycloalkyl wherein one or two of the carbon atoms of said cycloalkyl rings that contain at least 5 ring members may optionally and independently be replaced by an oxygen or sulfur atom or by NZ⁴ wherein Z⁴ Is hydrogen, C₁-C₄ alkyl or benzyl; and wherein each of the foregoing R⁵ groups is substituted with from one to four substituents R¹² wherein one to three of said substituents may be selected, independently, from chloro, C₁-C₆ alkyl and -O(C₁-C₆ alkyl) and one of said substituents may be selected from bromo, iodo, formyl, -CN, -CF₃, -NO₂, -NH₂, -NH(C₁-C₄ alkyl), -N(C₁-C₂ alkyl)(C₁-C₆ alkyl), -C(=O)O(C₁-C₄ alkyl), -C(=O)(C₁-C₄ alkyl), -COOH, -SO₂NH(C₁-C₄ alkyl), -SO₂N(C₁-C₂ alkyl)(C₁-C₄ alkyl), -SO₂NH₂, -NHSO₂(C₁-C₄ alkyl), -S(C₁-C₆ alkyl) and -SO₂(C₁-C₆ alkyl), and wherein each of the C₁-C₄ alkyl and C₁-C₆ alkyl moieties in the foregoing R⁵ groups may optionally be substituted with one or two substituents independently selected from fluoro, hydroxy, amino, methylamino, dimethylamino and acetyl;
R⁷ is hydrogen, C₁-C₄ alkyl, halo (e.g., chloro, fluoro, iodo or bromo), hydroxy, -O(C₁-C₄ alykl), -C(=O)(C₁-C₄ alkyl), -C(=O)O(C₁-C₄ alkyl), -OCF₃, -CF₃, -CH₂OH or -CH₂O(C₁-C₂ alkyl);
R¹⁰ is hydrogen, hydroxy, methoxy or fluoro;
R¹¹ is hydrogen or C₁-C₄ alkyl; and
Z is NH, oxygen, sulfur, -N(C₁-C₄ alkyl), -NC(=O)(C₁-C₂ alkyl), NC(=O)O(C₁-C₂alkyl) or CR¹³R¹⁴ wherein R¹³ and R¹⁴ are independently selected from hydrogen, trifluoromethyl and methyl with the exception that one of R¹³ and R¹⁴can be cyano;
with the proviso that: (a) in the five membered rings of structure I, there can not be two double bonds adjacent to each other; and (b) when R⁴ is attached to nitrogen, it is not halo, cyano or nitro.

Examples of more specific embodiments of formula I are the following, wherein A, B, G, Z, R³, R⁴ and R⁵ are defined as above, X is NR⁴, O, S or CR⁴ and R²⁵ is hydrogen, (C₁-C₄)alkyl or CF₃. 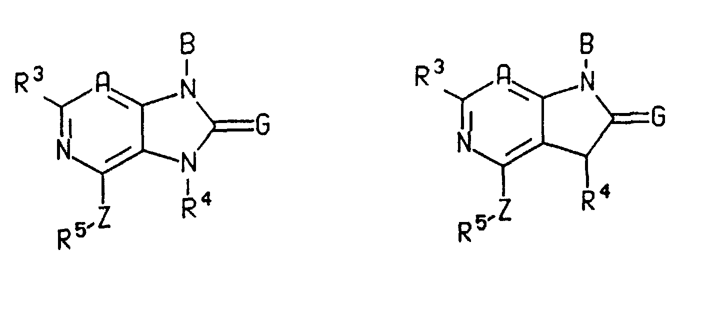 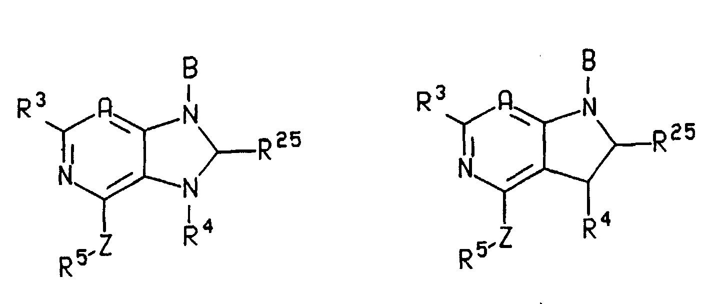 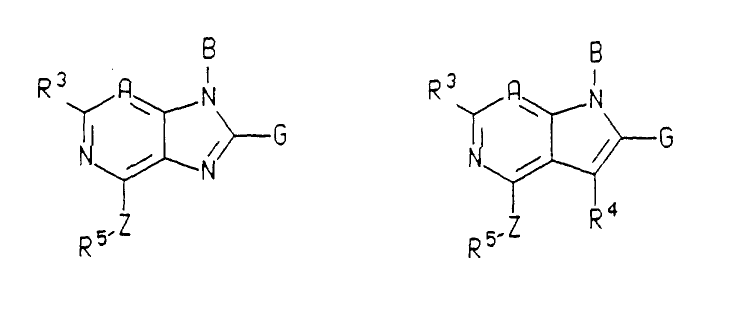 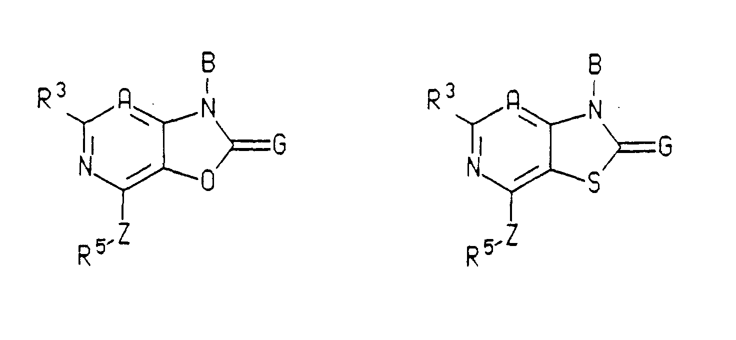 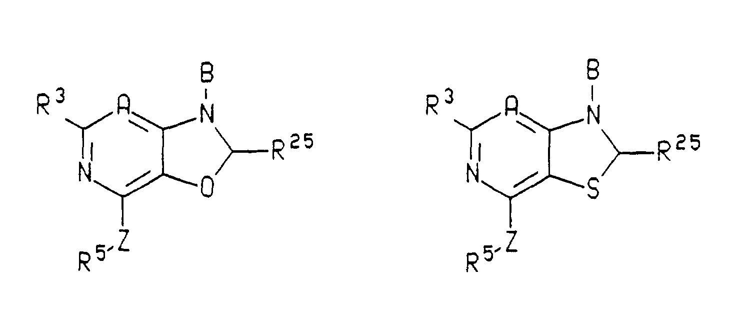 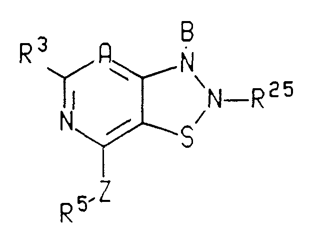 The compounds of formula I may contain one or more chiral centers and may therefore occur in different isomeric forms. The invention includes all stereoisomers and diastereomers of such compounds of formula I including racemic and optically active mixtures thereof.

This invention also relates to the pharmaceutically acceptable acid and base addition salts of compounds of the formula I . Examples of such pharmaceutically acceptable acid addition salts are the salts of hydrochloric acid, p-toluenesulfonic acid, maleic acid, fumaric acid, citric acid, succinic acid, salicylic acid, oxalic acid, hydrobromic acid, phosphoric acid, methanesulfonic acid, tartaric acid, di-p-toluoyl tartaric acid, and mandelic acid. Examples of such pharmaceutically acceptable base addition salts are the salts of the alkali metals and alkaline earth metals.

More specific embodiments of this invention include compounds of the above formula I wherein: R¹ is C₁-C₆ alkyl, which may optionally be substituted with one hydroxy, fluoro, CF₃, or C₁-C₄ alkoxy group and may optionally contain one double or triple bond; and R² is benzyl, C₁-C₆ alkyl, which may optionally contain one double or triple bond, wherein said C₁-C₆ alkyl and the phenyl moiety of said benzyl may optionally be substituted with one fluoro, CF₃, C₁-C₂ alkyl, C₁-C₂ alkoxy or chloro group.

Other more specific embodiments of the invention include compounds of formula I wherein R³ is methyl, ethyl, chloro or methoxy; R⁴ is methyl, ethyl or trifluoromethyl; G is hydrogen, methyl, ethyl, or E=G is C=O, C=S; R⁵ is phenyl, pyridyl, pyrimidyl which is substituted with more than two substituents independently selected from C₁-C₄ alkyl, -O(C₁-C₄ alkyl), (C₁-C₄ alkyl)-O-(C₁-C₄ alkyl), CF₃, OCF₃, -CHO, (C₁-C₄ alkyl)-OH, CN, Cl, F, Br, I and NO₂, wherein each of the foregoing (C₁-C₄) alkyl groups may optionally contain one double or triple bond.

Other more specific embodiments of the invention include compounds of the formula I wherein A is N, CH or CMe.

Examples of preferred compounds of this invention are:
2,5,6-trimethyl-7-(1-propylbutyl)-4-(2,4,6-trimethylphenoxy)-7H-pyrrolo[2,3-d]pyrimidine;
1-(1-ethylpropyl)-6-methyl-4-(2,4,6-trimethylphenylamino)-1,3-dihydro-imidazo[4,5-c]pyridin-2-one;
9-(1-ethylpropyl)-2-methyl-6-(2,4,6-trimethylphenylamino)-7,9-dihydro-purin-8-one;
1-(1-ethylpropyl)-6-methyl-4-(2,4,6-trimethylphenoxy)-1,3-dihydro-imidazo[4,5-c]pyridin-2-one;
1-(1-ethylpropyl)-6-methyl-(2,4,6-trimethylphenoxy)-1H-imidazo[4,5-c]pyridine;
1-(1-ethylpropyl)-3,6-dimethyl-4-(2,4,6-trimethylphenoxy)-1,3-dihydro-imidazo[4,5-c]pyridin-2-one; and
1-(1-ethylpropyl)-3,6-dimethyl-4-(2,4,6-trimethylphenylamino)-1,3-dihydroimidazo[4,5-c]pyridin-2-one.

Examples of other compounds of this invention are:
[1-(1-ethylpropyl)-2,6-dimethyl-1H-pyrrolo[3,2-c]pyridin-4-yl]-(2,4,6-trimethylphenyl)amine;
[1-(1-ethylpropyl)-3,6-dimethyl-1H-pyrrolo[3,2-c]pyridin-4-yl]-(2,4,6-trimethylphenyl)amine;
[1-(1-ethylpropyl)-6-methyl-1H-pyrrolo[3,2-c]pyridin-4-yl]-(2,4,6-trimethylphenyl)-amine;
[1-(1-ethylpropyl)-6-methyl-4-(2,4,6-trimethylphenoxy)-1H-pyrazolo[4,3-c]pyridine;
[1-(1-ethylpropyl)-3,6-dimethyl-4-(2,4,6-trimethylphenoxy)-1H-pyrazolo [4,3-c]pyridine;
[1-(1-ethylpropyl)-3,6-dimethyl-1H-pyrazolo[4,3-c]pyridin-4-yl]-(2,4,6-trimethylphenyl)amine;
[1-(1-ethylpropyl)-6-methyl-1 H-pyrazolo[4,3-c]pyridin-4-yl]-(2,4,6-trimethylphenyl)-amine;
N3,N3-diethyl-[5-methyl-N7-(2,4,6-trimethylphenyl)-isoxzolo[5,4-c]pyridin-3,7-diamine;
1-(1-ethylpropyl)-6-methyl-4-(2,4,6-trimethylphenoxy)-1H-[1,2,3]triazolo[4,5-c]pyridine;
1-(1-ethylpropyl)-6-methyl-4-(2,4,6-trimethylphenylsulfanyl)-1H-[1,2,3]triazolo[4,5-c]pyridine;
3-(1-ethylpropyl)-1,5-dimethyl-7-(2,4,6-trimethylbenzyl)-1 H-pyrrolo[2,3-c]pyridine;
3-(1-ethylpropyl)-1,5-dimethyl-7-(2,4,6-trimethylbenzyl)-1H-pyrrolo[3,2-d]pyrimidine;
5-(1-ethylpropyl)-3,6-dimethyl-1-(2,4,6-trimethylphenoxy)-pyrrolo[1,2-c]pyridine;
1-(1-ethylpropyl)-6-methyl-4-(2,4,6-trimethylphenoxy)-1H-[1 ,2,3]triazolo[4,5-c]pyridine;
[1-(ethylpropyl)-3,7-dimethyl-imidazo[1,5-c]pyrimidin-5-yl]-(2,4,6-trimethylphenyl)amine;
7-Bromo-1-(1-ethyl-propyl)-6-methyl-4-(2,4,6-trimethyl-phenoxy)-1H-[1,2,3]triazolo[4,5-c]pyridine;
1-(1-Ethyl-propyl)-6,7-dimethyl-4-(2,4,6-trimethyl-phenoxy)-1 H-[1,2,3]triazolo[4,5-c]pyridine;
1-(1-Ethyl-propyl)-6-methyl-4-(2,4,6-trimethyl-phenoxy)-1,3-dihydropyrrolo-[3,2-c]pyridin-2-one;
1-(1-Ethyl-propyl)-6-methyl-4-(2,4,6-trimethyl-phenoxy)-1 H-pyrrolo[3,2-c]pyridine;
1-(1-Ethyl-propyl)-6-methyl-4-(2,4,6-trimethyl-phenoxy)-2,3-dihydro-1H-pyrrolo [3,2-c]pyridine;
1-(1-Ethyl-propyl)-6-methyl-4-(2,4,6-trimethyl-phenoxy)-1H-imidazo[4,5-c]pyridin-2-ylamine;
1-(1-Ethyl-propyl)-3,6-dimethyl-4-(2,4,6-trimethyl-phenoxy)-1,3-dihydropyrrolo [3,2-c]pyridin-2-one;
1-(1-Ethyl-propyl)-3,3,6-trimethyl-4-(2,4,6-trimethyl-phenoxy)-1,3-dihydro-pyrrolo[3,2-c]pyridin-2-one;
1-(1-Ethyl-propyl)-3,3,6-trimethyl-4-(2,4,6-trimethyl-phenoxy)-2,3-dihydro-1 H-pyrrolo[3,2-c]pyridine;
1-(1-Ethyl-propyl)-3,6-dimethyl-4-(2,4,6-trimethyl-phenoxy)-1 H-pyrrolo [3,2-c]pyridine;
1-(1-Ethyl-propyl)-2-methoxy-3,6-dimethyl-4-(2,4,6-trimethylphenoxy)-1 H-pyrrolo[3,2-c]pyridine;
[1-(1-Ethyl-propyl)-6-methyl-1H-[1,2,3]triazolo[4,5-c]pyridin-4-yl]-(2,4,6-trimethylphenyl)-amine;
4-(4-Bromo-2,6-dimethyl-phenoxy)-1-(1-ethyl-propyl)-6-methyl-1H-oxazolo[5,4-c]pyridin-2-one;
1-(1-Ethyl-propyl)-6-methyl-4-(2,4,6-trimethyl-phenoxy)-1 H-oxazolo[5,4-c]pyridin-2-one;
1-(1-Ethyl-propyl)-3,6-dimethyl-4-(4-chloro-2,6-dimethyl-phenoxy)-1H-pyrrolo[3,2-c]pyridine;
1-(1-Ethyl-propyl)-3,6-dimethyl-4-(4-bromo-2,6-dimethyl-phenoxy)-1H-pyrrolo[3,2-c]pyridine;
1-(1-Ethyl-propyl)-3,6-dimethyl-4-(4-i-propyl-2,6-dimethyl-phenoxy)-1 H-pyrrolo [3,2-c]pyridine;
1-(1-Ethyl-propyl)-3,6-dimethyl-4-(4-t-butyl-2,6-dimethyl-phenoxy)-1H-pyrrolo[3,2-c]pyridine;
1-(1-Ethyl-propyl)-3,6-dimethyl-4-(4-ethyl-2,6-dimethyl-phenoxy)-1H-pyrrolo[3,2-c]pyridine;
1-(1-Ethy)-propyl)-3,6-dimethy)-4-(4-propyl-2,6-dimethyl-phenoxy)-1 H-pyrrolo[3,2-c]pyridine;
1-(1-Ethyl-propyl)-3,6-dimethyl-4-(4-trifluoro-2,6-dimethyl-phenoxy)-1H-pyrrolo[3,2-c]pyridine;
1-(1-Ethyl-propyl)-3,6-dimethyl-4-(4-methoxymethyl-2,6-dimethyl-phenoxy)-1H-pyrrolo[3,2-c]pyridine;
1-(1-Ethyl-propyl)-3,6-dimethyl-4-(4-hydroxymethyl-2,6-dimethyl-phenoxy)-1H-pyrrolo[3,2-c]pyridine;
1-(1-Ethyl-propyl)-3,6-dimethyl-4-(4-formyl-2,6-dimethyl-phenoxy)-1H-pyrrolo[3,2-c]pyridine;
1-(1-Ethyl-propyl)-3,6-dimethyl-4-(2-bromo-4-i-propyl-phenoxy)-1H-pyrrolo[3,2-c]pyridine;
1-(1-Ethyl-propyl)-3,6-dimethyl-4-(2,4-dimethyl-phenoxy)-1H-pyrrolo[3,2-c]pyridine;
1-(1-Ethyl-propyl)-3-ethyl-6-methyl-4-(2,6-dimethyl-4-chloro-phenoxy)-1H-pyrrolo[3,2-c]pyridine;
2-[4-(4-Chloro-2,6-dimethyl-phenoxy)-3,6-dimethyl-pyrrolo[3,2-c]pyridin-1-yl]-butan-1-ol;
2-[4-(4-bromo-2,6-dimethyl-phenoxy)-3,6-dimethyl-pyrrolo[3,2-c]pyridin-1-yl]-butan-1-ol;
2-[4-(4-i-propyl-2,6-dimethyl-phenoxy)-3,6-dimethyl-pyrrolo[3,2-c]pyridin-1-yl]-butan-1-ol;
2-[4-(4-Ethyl-2,6-dimethyl-phenoxy)-3,6-dimethyl-pyrrolo[3,2-c]pyridin-1-yl]-butan-1-ol;
2-[4-(4-trifluoromehtyl-2,6-dimethyl-phenoxy)-3,6-dimethyl-pyrrolo[3,2-c]pyridin-1-yl]-butan-1-ol;
2-[4-(2-bromo-4-i-propyl-phenoxy)-3,6-dimethyl-pyrrolo[3,2-c]pyridin-1-yl]-butan-1-ol.

Whenever reference is made herein to C₁-C₈ alkyl, a straight or branched chain alkyl of one to six carbon atoms is meant, such as methyl, ethyl, isopropyl, t-butyl or hexyl.

Whenever R² or R⁵ is a heterocyclic group, attachment of the group is through a carbon atom.

Whenever reference is made herein to C₁-C₄ alkyl or C₁-C₆ alkyl which 'may contain one double or triple bond" in the definitions of R¹ and R⁴, it is understood that at least two carbons are present in the alkyl for one double or triple bond.

Whenever reference is made herein to halo or halogen, fluoro, chloro, bromo or iodo is meant unless indicated otherwise.

This invention also relates to a pharmaceutical composition for the treatment or prevention of (a) a disorder, the treatment of which can be effected or facilitated by antagonizing CRF, including but not limited to disorders induced or facilitated by CRF, or (b) a disorder selected from inflammatory disorders such as rheumatoid arthritis and osteoarthritis, pain, asthma, psoriasis and allergies; generalized anxiety disorder; panic; phobias; obsessive-compulsive disorder; post-traumatic stress disorder; sleep disorders induced by stress; pain perception such as fibromyalgia; mood disorders such as depression, including major depression, single episode depression, recurrent depression, child abuse induced depression, and postpartum depression; dysthemia; bipolar disorders; cyclothymia; fatigue syndrome; stress-induced headache; cancer; human immunodeficiency virus (HIV) infections; neurodegenerative diseases such as Alzheimer's disease, Parkinson's disease and Huntington's disease; gastrointestinal disorders such as ulcers, irritable bowel syndrome, Crohn's disease, spastic colon, diarrhea, and post operative ilius and colonic hypersensitivity associated with psychopathological disturbances or stress; eating disorders such as anorexia and bulimia nervosa; hemorrhagic stress; chemical dependencies and addictions (e.g., dependencies on alcohol, cocaine, heroin, benzodiazepines, or other drugs); drug and alcohol withdrawal symptoms; stress-induced psychotic episodes; euthyroid sick syndrome; syndrome of inappropriate antidiarrhetic hormone (ADH); obesity; infertility; head traumas; spinal cord trauma; ischemic neuronal damage (e.g., cerebral ischemia such as cerebral hippocampal ischemia); excitotoxic neuronal damage; epilepsy; cardiovascular and heart related disorders including hypertension, tachycardia and congestive heart failure; stroke; immune dysfunctions including stress induced immune dysfunctions (e.g., stress induced fevers, porcine stress syndrome, bovine shipping fever, equine paroxysmal fibrillation, and dysfunctions induced by confinement in chickens, sheering stress in sheep or human-animal interaction related stress in dogs); muscular spasms; urinary incontinence; senile dementia of the Alzheimer's type; multiinfarct dementia; amyotrophic lateral sclerosis; osteoporosis; psychosocial dwarfism; and hypoglycemia in a mammal, including a human, comprising an amount of a compound of the formula I or a pharmaceutically acceptable salt thereof, that is effective in the treatment or prevention of such disorder, and a pharmaceutically acceptable carrier.

This invention also relates to a pharmaceutical composition for the prevention or premature births in a mammal, including a human, comprising an amount of a compound of the formula I or a pharmaceutically acceptable salt thereof, that is effective in the prevention of such disorder, and a pharmaceutically acceptable carrier.

This invention further includes the use of a compound of the formula I, or a pharmaceutically aceptable salt thereof for the preparation of a medicament for the treatment of (a) a disorder, the treatment of which can be effected or facilitated by antagonizing CRF, including but not limited to disorders induced or facilitator by CRF, or (b) a disorder , selected from inflammatory disorders such as rheumatoid arthritis and osteoarthritis, pain, asthma, psoriasis and allergies; generalized anxiety disorder; panic; phobias; obsessive-compulsive disorder; post-traumatic stress disorder; sleep disorders induced by stress; pain perception such as fibromyalgia; mood disorders such as depression, including major depression, single episode depression, recurrent depression, child abuse induced depression, and postpartum depression; dysthemia; bipolar disorders; cyclothymia; fatigue syndrome; stress-induced headache; cancer; human immunodeficiency virus (HIV) infections; neurodegenerative diseases such as Alzheimer's disease, Parkinson's disease and Huntington's disease; gastrointestinal diseases such as ulcers, irritable bowel syndrome, Crohn's disease, spastic colon, diarrhea, and post operative ilius and colonic hypersensitivity associated with psychopathological disturbances or stress; eating disorders such as anorexia and bulimia nervosa; hemorrhagic stress; stress-induced psychotic episodes; euthyroid sick syndrome; syndrome of inappropriate antidiarrhetic hormone (ADH); obesity; infertility; head traumas; spinal cord trauma; ischemic neuronal damage (e.g., cerebral ischemia such as cerebral hippocampal ischemia); excitotoxic neuronal damage; epilepsy; cardiovascular and heart related disorders including hypertension, tachycardia and congestive heart failure; stroke; immune dysfunctions including stress induced immune dysfunctions (e.g., stress induced fevers, porcine stress syndrome, bovine shipping fever, equine paroxysmal fibrillation, and dysfunctions induced by confinement in chickens, sheering stress in sheep or human-animal interaction related stress in dogs); muscular spasms; urinary incontinence; senile dementia of the Alzheimer's type; multiinfarct dementia; amyotrophic lateral sclerosis; chemical dependencies and addictions (e.g., dependencies on alcohol, cocaine, heroin, benzodiazepines, or other drugs); drug and alcohol withdrawal symptoms; osteoporosis; psychosocial dwarfism and hypoglycemia in a mammal, including a human.

### Detailed Description of the Invention

The following compounds having the formulas IV, V and VI are useful as starting materials and intermediates in the synthesis of compounds of the formula I.

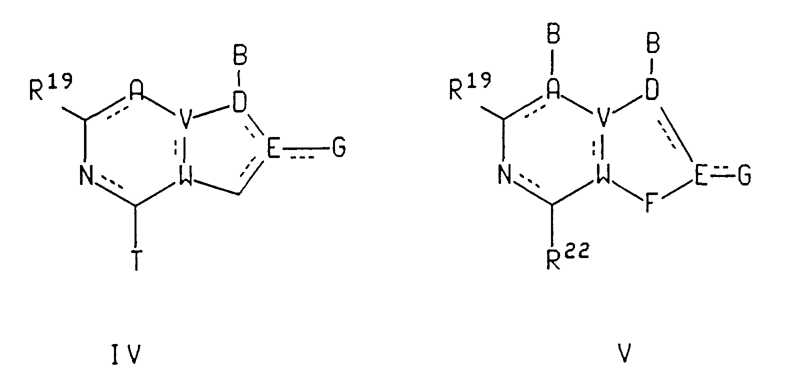

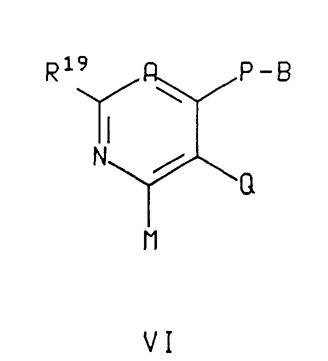

In the above compounds of formulas IV, V and VI, R¹⁹ is (C₁-C₄)alkyl, fluoro, chloro, bromo or iodo, T is chloro, bromo, iodo, -OCOCF₃ or -OSO₂CF₃, M is T or ZR⁵, R²² is OH or NH₂, P is NH, CHCN or CHCOO(C₁-C₄ alkyl), Q is -NH₂, -CH₂COO(C₁-C₄alkyl), CH₂CN, -OH or -SH, V and W are C and A, B, D, E, F and G are defined as above.

Methods of preparing the compounds and compositions of this invention are described below. In the discussion and reaction schemes that follow, R¹ through R⁵, R⁷ through R¹⁴, R¹⁹, R²⁵ A, B, D, E, F, G, X, the dashed lines and structural formulas I, II, III, IV, V and VI, unless otherwise indicated, are defined as above.

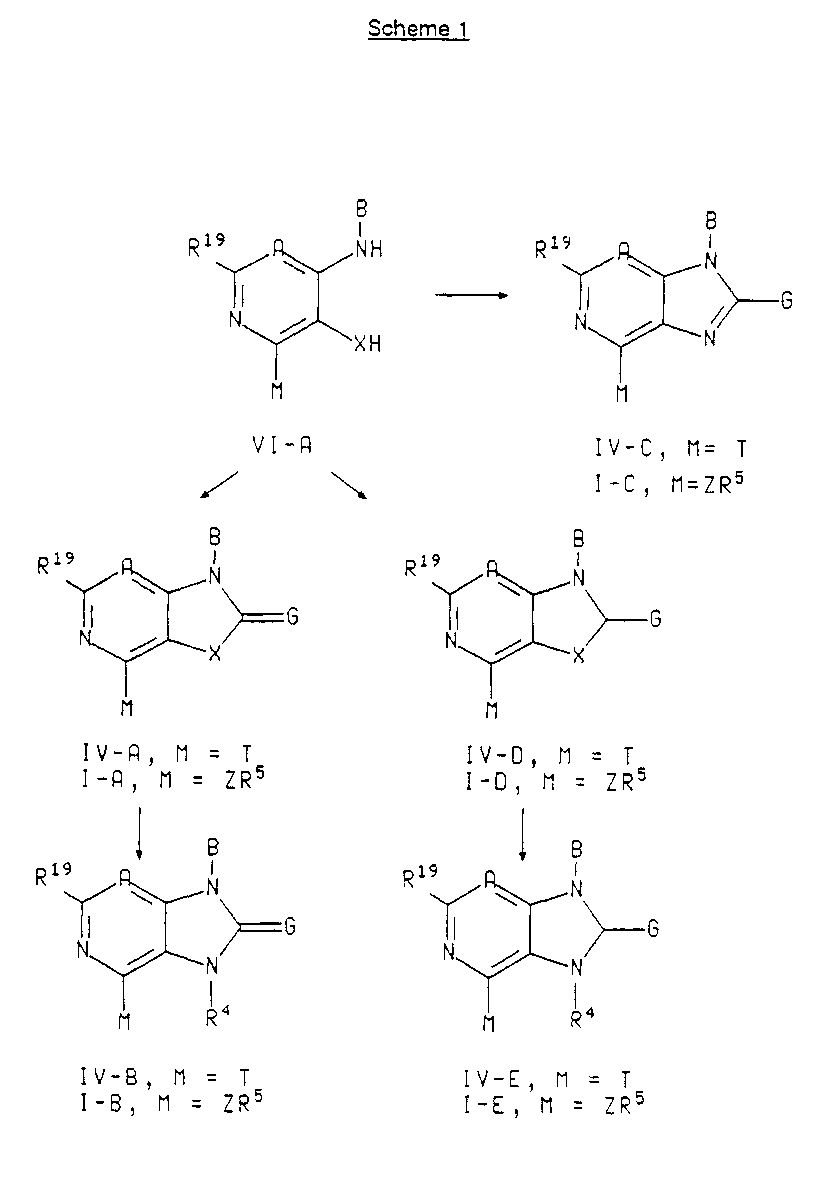

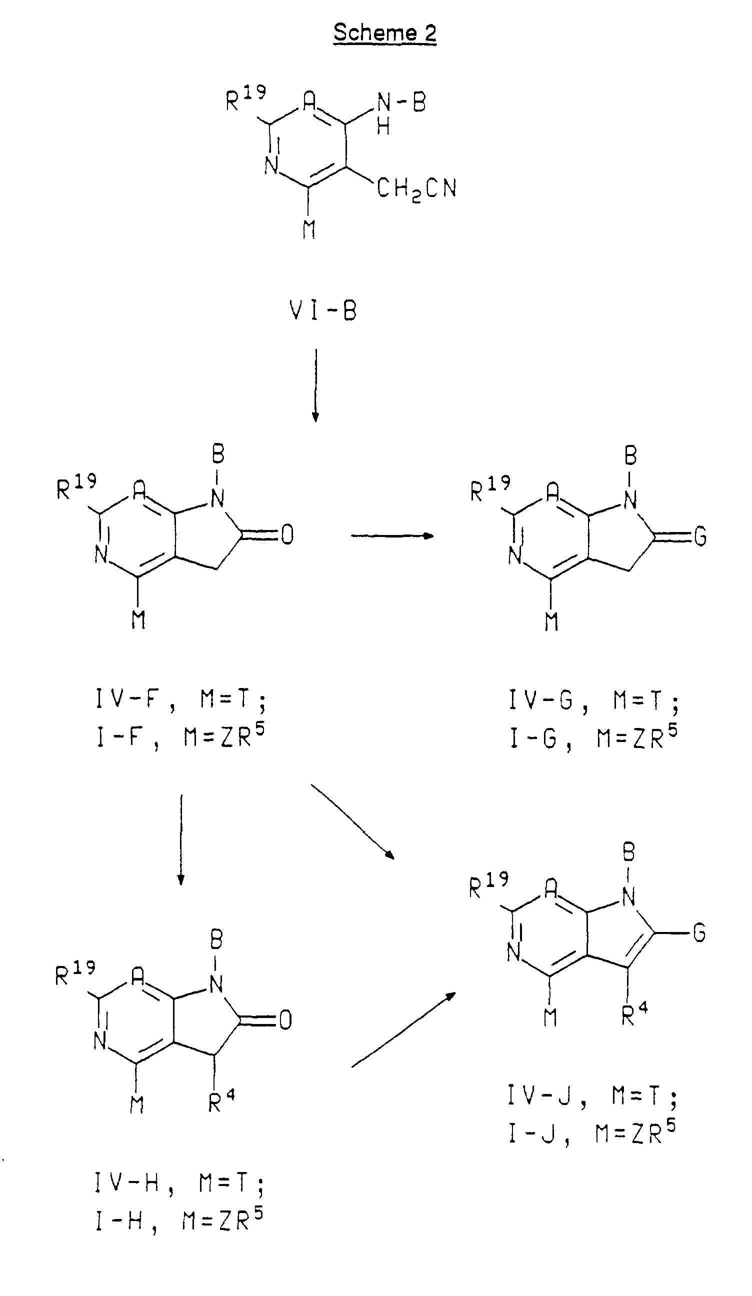

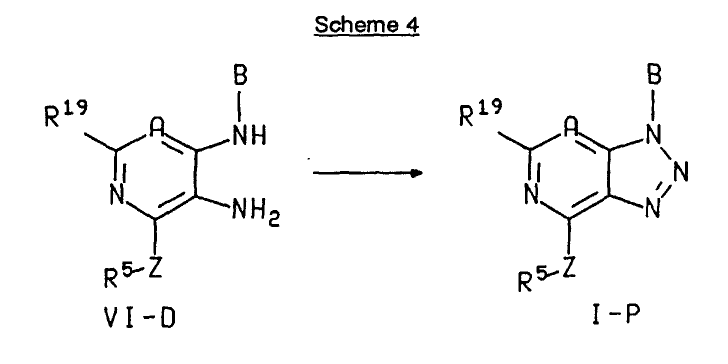

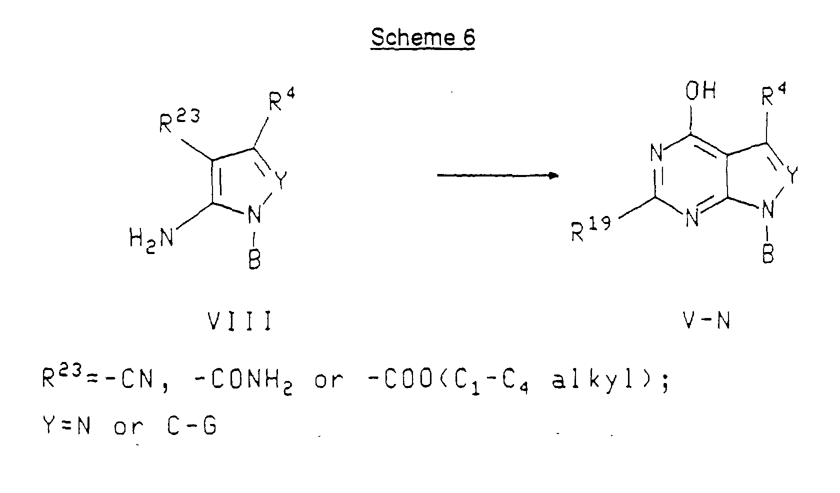

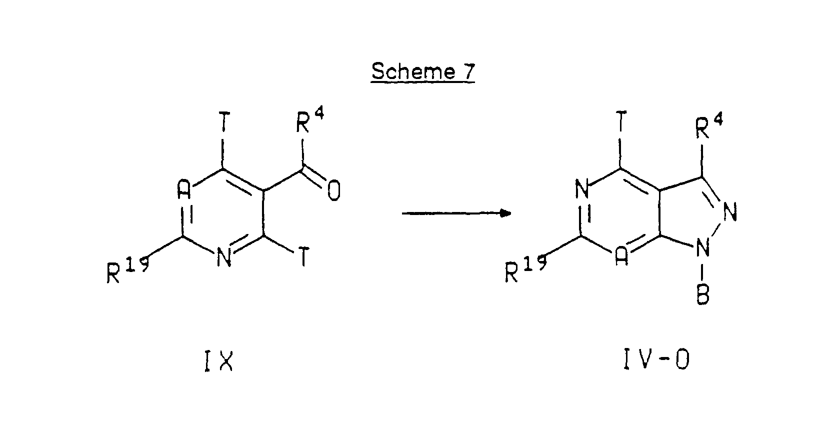

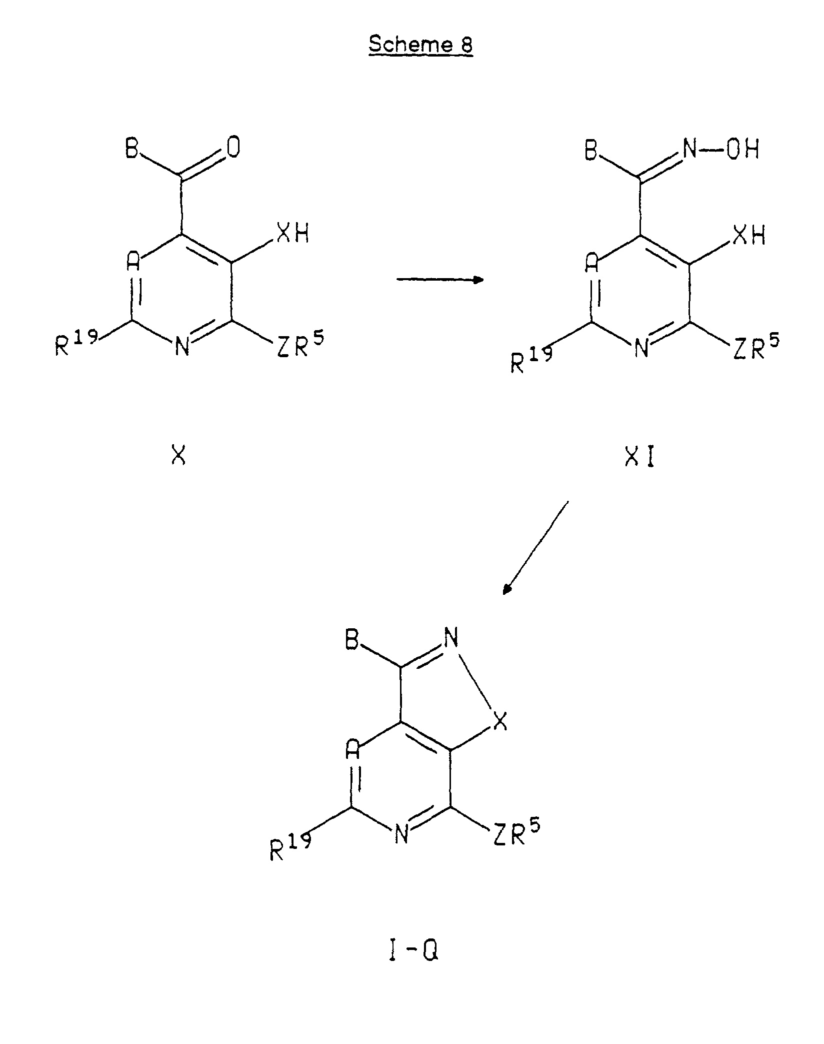

Compounds of formula I wherein R³ is C₁-C₄alkyl, fluoro, chloro, bromo, or iodo (hereinafter R¹⁹) may be prepared by reaction of a compound of formula IV, wherein T is Cl, Br, I, -O-COCF₃, -OSO₂CF₃, V and W are C and A, T, D, E, F, and G are defined as above with reference of formula I with a compound of formula R⁵ZH wherein Z and R⁵ are as defined above. This reaction is generally carried out with or without a solvent, in the presence of a base, at a temperature from about 0°C to about 270°C, and at a pressure between about 1 atmosphere and 300 psi. Suitable solvents include organic solvents such as tetrahydrofuran (THF), acetonitrile, dimethylsulfoxide (DMSO), acetone, C₂-C₁₅ alcohols, chloroform, dioxane, chlorobenzene, benzene, toluene, xylene, sulfolane, pyridine, quinoline, 2,4,6-trimethylpyridine, acetamide, di-(C₁-C₂)alkylacetamide, or 1-methyl-2-pyrrolidinone (NMP).

When Z is NH, an excess of R⁵ZH may be used both as a reagent and as a base. Examples of bases other than R⁵ZH that may be used include potassium carbonate, sodium hydride, potassium hydride, sodium (C₁-C₄) alkoxides, potassium (C₁-C₄) alkoxides, sodium, sodium amide, tri-[(C₁-C₆) alkyl]amines, organolithium or organosodium compounds such as n-butyllithium, s-butyllithium, t-butyllithium, lithium diisopropylamide, lithium bis(trimethylsilyl)amide, sodium diisopropylamide or sodium bis(trimethylsilyl)amide, and organometallic bases such as Grignard reagents. This reaction is generally carried out in an appropriate solvent (e.g., THF, dioxane, sulfolane, DMSO or NMP, with or without an additinal catalyst such as a copper halide, oxide or sulfate (e.g., Cul, CuBr, Cu₂O, CuCI, CuSO₄ Cul_{2,} CuBr₂, CuCl₂ or Cu(O)), a Pd(0) salt such as tetrakis(triphenylphosphine)palladium (Pd(PPH₃)₄), a Pd(II) salt such as palladium diacetate (Pd(OAc)₂) or racemic or (R)- or (S)-2,2'-bis(diphenylphosphino)-1,1 '-binaphthyl (BINAP), at temperature from about room temperature to about 270°C.

When Z is O or S, a base which is capable of deprotonating R⁵ZH may be used, such as potassium carbonate, sodium carbonate, sodium, sodium amide, an alkali metal hydride such as sodium or potassium hydride, a sodium C₁-C₄ alkoxide, a potassium C₁-C₄ alkoxide, sodium amide, a tri-(C₁-C₆alkyl)amine or an organometallic base such as n-butyllithium, s-butyllithium, t-butyllithium, lithium diisopropylamide, lithium bis(trimethylsilyl)amide, sodium diisopropylamide or sodium bis(trimethylsilyl)amide. The reaction temperature can range from about 0°C to about 180°C and is preferably from about 50°C to about 140°C. Suitable solvents include DMSO, THF, sulfolane, dioxane and NMP.

When Z is CHCN or CHCOO(C₁-C₄ alkyl), a base that is capable of deprotonating R⁵ZH may be used, such as an alkali metal hydride (e.g., sodium or potassium hydride), a sodium C₁-C₄ alkoxide or an organometallic base such as n-butyllithium, s-butyllithium, t-butyllithium, lithium diisopropylamide, lithium bis(trimethylsilyl)amide, sodium diisopropylamide or sodium bis(trimethylsilyl)amide, in an appropriate solvent, e.g., a solvent selected from THF, DMSO, dioxane, methylene chloride (CH₂Cl₂), chloroform (CHCl₃), toluene, xylene, benzene and C₁-C₆ alkanols.

Compounds of the formula I wherein Z is CR¹³CN, CHR¹³, N(C₁-C₄ alkyl), NC(=O)(C₁-C₂ alkyl) and NC(=O)O(C₁-C₂ alkyl) may be prepared as described below, using methods that are well known in the art.

When Z is CR¹³CN, compounds of formula I may be prepared by reaction of the corresponding compounds wherein Z is CHCN with a base such as an alkali metal hydride such as sodium or potassium hydride, n-butyllithium, s-butyllithium, t-butyllithium, lithium diisopropylamide, lithium bis(trimethylsilyl)amide or sodium diisopropylamide, followed by reacting with a compound of the formula R¹³L wherein L is a leaving group such as I, Br, Cl, mesylate (OMs) or tosylate (OTs).

Compounds of the formula I wherein Z is CHR¹³ may be prepared by acid hydrolysis (using, e.g., 85% phosphoric acid) of the corresponding compounds wherein Z is CR¹³CN, followed by decarboxylation upon heating. Further alkylation in the presence of base and a compound of the formula and R¹⁴L, wherein L is defined as above, will yield the corresponding compounds of formulas I, II and III wherein Z is CR¹³R¹⁴.

When Z is N(C₁-C₄ alkyl), compounds of the formula I may be prepared by reaction of the corresponding compounds wherein Z is NH with a base, followed by reaction with a compound of the formula (C₁-C₄ alkyl)-L, wherein L is defined as above. Bases such as lithium diisopropylamide, lithium bis(trimethylsilyl)amide, sodium diisopropylamide may also be used.

When Z is NC(=O)(C₁-C₂ alkyl) or NC(=O)O(C₁-C₂ alkyl), compounds of the formula I may be prepared by reaction of the corresponding compounds wherein Z is NH with a compound of the formula [(C₁-C₂ alkyl)-C(=O)]₂O, (C₁-C₂ alkyl)-C(=O)(Cl) or (C₁-C₂ alkyl)-O-C(=O)(Cl) in the presence of base such as a tri(C₁-C₆ alkyl)amine or pyridine.

Compounds of formula I wherein Z and R⁵ are defined with reference formula I above and R³ is -O-(C₁-C₄ alkyl) or -S-(C₁-C₄ alkyl) (hereinafter R²⁰), may be prepared by reacting the corresponding compounds of the formula I wherein R³ is chloro, bromo, OTs or iodo, with a nucleophile of the formula R²⁰H, wherein R²⁰H is an alkanol or an alkane thiol, optionally in the presence of an organic or inorganic base. Suitable bases include sodium, sodium hydride, potassium hydride, lithium diisopropylamide, lithium bis(trimethylsilyl)amide and sodium diisopropylamide.

Compounds of the formulas I wherein R³ is fluoro may be prepared by reacting the corresponding compounds wherein R³ is chloro, bromo, iodo, -OCOCF₃, or -OSO₂CF₃ with tetrabutylammonium fluoride, potassium fluoride or another fluoride agent, using procedures well known to those skilled in the art. Compounds of the formulas I wherein R³ is CN may be prepared by reacting the corresponding compounds of formulas I, II, or III wherein R³ is chloro, bromo, iodo, -OCOCF₃, or -OSO₂CF₃ with sodium cyanide, potassium cyanide, copper cyanide or other cyanide agent, using methods well known to those of skill in the art.

When R²² is OH, compounds of formula IV may be prepared from compounds of formula V. When T is Cl, the compound of formula IV may be prepared by heating a compound of formula V with an excess of POCl₃, POCl₃/PCl₅ or PCl₅ at a temperature from about 80°C to about 150°C, preferably at about the reflux temperature. When T is Cl, Br, or I, the compound of formula IV may be prepared by reacting the corresponding compound of formula IV wherein T is -OCOCF₃ or -OSO₂CF₃, preferably -OSO₂CF₃, with a sodium, potassium, or lithium halide in a suitable solvent such as sulfolane, DMSO or 1-methyl-2-pyrrolidinone. Compounds of formula IV wherein T is -OCOCF₃ or -OSO2CF₃ may be prepared by reacting a compound of formula V with (CF₃CO)₂O, (CF₃SO₂)₂O, CF₃SO₂Cl, or CF₃COCl, with or without a base. Suitable bases include tri-(C₁-C₆ alkyl)amines and sodium and potassium carbonates. When R³ is chloro, bromo, iodo, -OCOCF₃, or -OSO₂CF₃, it is preferable for R³ and T to be the same.

When R²² is NH₂, compounds of the formula IV may be prepared by reacting a compound of the formula V with a compound of the formula (C₁-C₄ alkyl)-O-N=O and a copper (II) halide in an appropriate solvent such as acetonitrile, acetone, toluene, methylene chloride or dichloroethane, at a temperature from about room temperature to about the reflux temperature. This reaction is preferably carried out in acetonitrile at the reflux temperature.

Alternatively, as shown in Scheme I, compounds of the formulas I-A, I-C and I-D may be prepared from compounds of the formula VI-A. Referring to Scheme 1, reaction of a compound of the formula VI-A (wherein M is T or ZR⁵, T is Cl, Br, I, OTs or -OCOCF₃, X is O, NH, NR⁴, or S, and A, B, and R¹⁹ are defined as above) with phosgene or its equivalent (e.g., diphosgene or triphosgene), thiophosgene, or CNBr, in the presence of a base such as a tri-(C₁-C₄) alkylamine or sodium hydride, in an appropriate solvent (e.g., methylene chloride, chloroform or THF) in the presence of a tri(C₁-C₄ alkyl)amine, will yield compounds of the formula IV-A wherein M is T and G is O, S, or NH, or the corresponding compounds of the formula I-A wherein M is ZR⁵. Compounds of formula I-C and IV-C may be prepared by heating compounds of formula VI-A with a compound of the formula (C₁-C₄ alkyl)-C-[O(C₁-C₂ alkyl)]₃ or HC[O-(C₁-C₂)alkyl]₃ in the presence of a catalytic amount of acid (e.g., p-toluene sulfonic, conc. sulfuric acid or gaseous hydrogen chloride), in an appropriate solvent such as toluene, benzene or xylene, under a Dean-Stark trap. Compounds of the formula I-D wherein G is hydrogen or C₁-C₄ alkyl may be prepared by heating a compound of the formula GCHO or GH(OMe)₂ in the presence of an acid catalyst. Alkylation of compounds of the formula I-A or I-D wherein X is NH with a compound of the formula R⁴L wherein L is a leaving group, as defined above, or wherein R⁴L is (C₁-C₄)₂SO₂, in the presence of a base that is capable of deprotonating NH such as sodium hydride or butyllithium, yields the corresponding alkylated derivative of the formula I-B or I-E, respectively. Compounds of formulas IV-A, IV-B, IV-C, IV-D and IV-E wherein M is T may be converted to the corresponding compounds of formulas I-A through I-E wherein M is ZR⁵ by the methods described above for converting compounds of the formula IV into compounds of the formula I

Compounds of the formula I-F may be prepared, as illustrated in Scheme 2, by reacting the corresponding compounds of the formula Vl-B (wherein M, X, A, B, and R¹⁹ are defined as in the preceding paragraph) with a base that is capable of deprotonating NH (such as sodium hydride, potassium hydride, or an organometallic base such as lithium diisopropylamide, lithium bis(trimethylsilyl)amide or sodium diisopropylamide) in an appropriate solvent, e.g., a solvent selected from THF, dioxane, DMSO, benzene, toluene, methylene chloride and chloroform. Alternatively, heating a compound of the formula Vl-B in the presence of an acid (e.g., p-toluenesulfonic acid, aqueous phosphoric acid concentrated sulfuric acid or gaseous hydrogen chloride), in an appropriate solvent such as toluene, benzene or xylene, will yield the corresponding compound of formula I-F. Alkylation of compounds of formula I-F with a compound of the formula R⁴L, defined as above, in the presence of a base such as sodium hydride, potassium hydride, or an organometallic base such as lithium diisopropylamide, lithium bis(trimethylsilyl)amide or sodium diisopropylamide, in an appropriate solvent such as THF or dioxane, yields the corresponding compounds of formula I-H.

Compounds of the formula I-J wherein G is chloro or trifliate may be prepared by heating the corresponding compounds of formula I-H with POCl₃, with or without PCl₅ or (Tf)₂O (wherein Tf is triflate), respectively. Displacement of the chloro or OTf group of a compound of formula I-G with a nucleophile will yield the corresponding compound of formula I-J wherein G is defined for formula I. Compounds of the formula I-G wherein G is S may be prepared by reacting the corresponding compounds of formula I-F with Lawessen's reagent or P₄S₁₀. Compounds of the formula I-J wherein G is H may be prepared by reduction of the corresponding compounds of formula I-F or I-H with lithium aluminum hydride (LiAlH₄) or borane methyl sulfide complex (BH₃•DMS), followed by acid hydrolysis. Organometallics addition (using, e.g., GLi, GMgBr or GMgl), followed by acid hydrolysis, employing methods well known in the art, will provide compounds of formula I-J wherein G is (C₁-C₄) alkyl.

Deprotonation of I-H with a base such as NaH in HMPA, followed by quenching with a (C₁-C₄ alkyl)₂SO₂- or C₁-C₄ alkyl containing electrophile, will yield a compound of formula I-J wherein G is O-(C₁-C₄ alkyl).

Compounds of the formula I-P may be prepared, as shown in Scheme 4, by reacting compounds of the formula VI-D with sodium nitrite in 48% hydrogen bromide in the presence of cuprous bromide or bromine at a temperature from about 0°C to about the reflux temperature. Preferably, the reaction is carried out at about 0°C for about thirty minutes, and then at mild reflux.

As shown in scheme 6, compounds of the formulas V-N, wherein Y is N or C(C₀ - C₄) alkyl, may be prepared by heating compounds of the formula VIII, wherein R²³ is CN, X is O, S, NH or N(C₁-C₄ alkyl), and Y is CH, N or C(C₁-C₄ alkyl), with a compound of formula acid (R²⁴CO)₂O in R²⁴COOH, at temperature from about 25°C to about 120°C, preferably at the reflux temperature of the reaction mixture. The above formed compounds wherein R¹⁹ is hydrogen, C₁-C₆ alkyl or hydroxy may be heated in aqueous acid to give compounds of formula V-N. Appropriate acids include 85% phosphoric acid , hydrochloric acid, sulfuric acid and acetic acid. Eighty-five percent phosphoric acid is preferred. The reaction is carried out at a temperature from about 25°C to about 180°C, preferably from about 100°C to about 150°C.

Compounds of the formulas V-N (wherein Y is N) may be prepared, as shown in Scheme 6, by heating compounds of the formula VIII, respectively, [wherein R²³ is CONH₂ or COO(C₁-C₄ alkyl), X is O, S, NH or N(C₁-C₄ alkyl) and Y is CH or C(C₁-C₄ alkyl)], with a compound of the formula C₁₉CONH₂ wherein R¹⁹ is as defined above. This reaction can be conveniently carried out in the absence of a solvent at temperatures ranging from about 100°C to about 250°C.

Compounds of formula IV-O may be prepared by reacting the corresponding compounds of formula IX wherein A, T, R¹⁹ and R⁴ are defined as above with BNHNH₂ in an appropriate solvent as shown in Scheme 7. Suitable solvents include C₁-C₅ alcohols, acetonitrile, toluene, chlorobenzene, xylene, toluene, dioxane, chloroform and methylene chloride, preferably in i-propanol or acetonitrile.

Compounds of the formula I-Q can be prepared as illustrated in Scheme 8. Compounds of formula Xl wherein B is CR¹R²R¹⁰ or CN, X is 0, S, NH, N(C₁-C₄ alkyl), and R¹⁰, A, Z, R⁵ are defined as above may be prepared by reacting compounds of formula X with hydroxylamine•HCl in a mixture of a solvent selected from C₁-C₅ alcohols, CH₃CN, acetone, dioxane and water, with or without sodium acetate, at a temperature from about room temperature to about 120°C, preferably at about the reflux temperature. Compounds of formula Xl can then be reacted with an appropriate agent convert the hydroxy group of the oxime into a good leaving group such as -OAc, -OCOCF₃, -OSO₂CF₃, -OSO₂CH₃ or -OSO₂C₆H₅CH₃ (p-tosylate). Examples of such appropriate agents are acetic anhydride, trifluoroacetic anhydride, triflic anhydride, methanesulfonyl chloride and p-toluenesulfonyl chloride. This reaction is generally conducted in an appropriate solvent such as methylene chloride, chloroform, acetonitrile, acetone, THF or pyridine, with or without a base such as N,N-dimethylpyridine or a tri-(C₁-C₈ alkyl) amine, at temperature from about 0°C to about 120°C, preferably from about room temperature to about 80°C. Most preferably, an excess of acetic anhydride is used at a temperature between 80°C and the reflux temperature. The resulting compounds can then be heated in an appropriate solvent such as DMF, DMSO, sulfolane, dioxane, THF or NMP in the presence of base such as pyridine, a tri(C₁-C₄ alkyl) amine or sodium hydride, at temperature from about 0°C to about 180°C, preferably from about room temperature to about 150°C, to give the final cyclized compounds of formula I-Q.

Compounds of formula I-Q wherein B is -CN can be converted into the corresponding compounds wherein B is NR¹R² or NHCR¹R²R¹⁰ using a Curtius rearrangement reaction, as described below. Compounds of formula I-Q wherein B is CN are subjected to acid hydrolysis with, e.g., aqueous phosphoric acid, at a temperature between about 80°C and about 150°C, to yield the corresponding compounds wherein B is COOH. Compounds of the formula I-Q wherein B is COOH can be converted into the corresponding compounds wherein B is -NH₂ by reacting them with diphenylphosphorylazide in t-butyl alcohol in the presence of a tri(C₁-C₄ alkyl) amine, followed by acid hydrolysis using, e.g., trifluoroacetic acid, according to procedures well known in the art. The amino derivatives so formed can be converted, also using standard methods well known in the art, into the corresponding compounds wherein B is NR¹R²R¹⁰ via an alkylation or reduction amination reaction. Such a procedure is described above for forming compounds of the formula IB.

Reaction of compounds of formula I-Q wherein B is CN with a Grignard reagent (e.g., R²MgX' wherein X' is halo) at a temperature from about 0°C to about room temperature in THF, ether or dioxane, followed by quenching with an acid, using the conditions well known in the art, will afford the corresponding ketones of formula I-Q wherein B is COR². Reduction of such ketones with sodium borohydride in a C₁-C₅ alkyl alcohol will afford the corresponding compounds of formula I-Q wherein B is CHR²OH. Alkylation of compounds of formula I-Q wherein B is CHR²OH with R¹-L (wherein L is a leaving group such as halo, mesylate or tosylate) in the presence of a base such as sodium hydride or potassium hydride will yield the corresponding compounds wherein B is CHR¹R². This reaction is typically carried in an appropriate solvent, e.g., THF, dioxane, ether, toluene or DMSO, at temperature between about 0°C and about 100°C, preferably between about 0°C and about room temperature.

The starting materials and intermediates of formulas IV, V, VI, VII, VIII, IX and X are commercially available, known in the art, or able to be synthesized using the procedures disclosed in PCT Patent Application PCT/IB95/00439, PCT Patent Application PCT/IB95/00373, U.S. Patent Application 08/481,413, U.S. Patent Application 08/448,539, and U.S. Patent Application 08/254,820, all of which are referred to and incorporated herein by reference in their entireties above.

In each of the above reactions, pressure is not critical. Pressures in the range of about 0.5-20 atm (0.5-20 bars) are suitable, and ambient pressure (generally, about one atmosphere) is preferred as a matter of convenience. Also, for those reactions where the preferred temperature varies with the particular compounds reacted, no preferred temperature is stated. For such reactions, preferred temperatures for particular reactants may be determined by monitoring the reaction using thin layer chromatography or gas chromatography/mass spectroscopy.

The preparation of other compounds of the formula I not specifically described in the foregoing experimental section can be accomplished using combinations or variations of the reactions described above that will be apparent to those skilled in the art.

Compounds of the formula I, that are basic in nature are capable of forming a wide variety of different salts with various inorganic and organic acids. Although such salts must be pharmaceutically acceptable for administration to animals, it is often desirable in practice to initially isolate a compound of the formulas I, II or III from the reaction mixture as a pharmaceutically unacceptable salt and then simply convert the latter back to the free base compound by treatment with an alkaline reagent, and subsequently convert the latter free base to a pharmaceutically acceptable acid addition salt. The acid addition salts of compounds of the formula I can be prepared in a conventional manner by treating a solution or suspension of the corresponding free base with one chemical equivalent of a pharmaceutically acceptable acid. Conventional concentration or crystallization techniques can be employed to isolate the salts. Illustrative of suitable acids are acetic, lactic, succinic, maleic, tartaric, citric, gluconic, ascorbic, benzoic, cinnamic, fumaric, sulfuric, phosphoric, hydrochloric, hydrobromic, hydroiodic, sulfamic, sulfonic acids such as methanesulfonic, benzene sulfonic, p-toluenesulfonic, and related acids.

Compounds of the formula I that are also acidic in nature, are capable of forming base salts with various pharmacologically acceptable cations. Examples of such salts include the alkali metal or alkaline-earth metal salts and particularly, the sodium and potassium salts. These salts are all prepared by conventional techniques. The chemical bases which are used as reagents to prepare the pharmaceutically acceptable base salts of this invention are those which form non-toxic base salts with the acidic compounds of formula I. Such non-toxic base salts include those derived from such pharmacologically acceptable cations as sodium, potassium calcium and magnesium, etc. These salts can easily be prepared by treating the corresponding acidic compounds with an aqueous solution containing the desired pharmacologically acceptable cations, and then evaporating the resulting solution to dryness, preferably under reduced pressure. Alternatively, they may also be prepared by mixing lower alkanolic solutions of the acidic compounds and the desired alkali metal alkoxide together, and then evaporating the resulting solution to dryness in the same manner as before. In either case, stoichiometric quantities of reagents are preferably employed in order to ensure completeness of reaction and maximum yields of the desired final product.

The active compounds of this invention may be administered alone or in combination with pharmaceutically acceptable carriers, in either single or multiple doses. Suitable pharmaceutical carriers include inert solid diluents or fillers, sterile aqueous solutions and various organic solvents. The pharmaceutical compositions formed by combining the novel compounds of formula I and their pharmaceutically acceptable carriers can then be readily administered in a variety of dosage forms such as tablets, powders, lozenges, syrups, injectable solutions and the like. These pharmaceutical compositions can, if desired, contain additional ingredients such as flavorings, binders, excipients and the like. Thus, for purposes of oral administration, tablets containing various excipients such as sodium citrate, calcium carbonate and calcium phosphate may be employed along with various disintegrants such as starch, methylcellulose, alginic acid and certain complex silicates, together with binding agents such as polyvinylpyrrolidone, sucrose, gelatin and acacia. Additionally, lubricating agents such as magnesium stearate, sodium lauryl sulfate and talc are often useful for tabletting purposes. Solid compositions of a similar type may also be employed as fillers in soft and hard filled gelatin capsules. Preferred materials for this include lactose or milk sugar and high molecular weight polyethylene glycols. When aqueous suspensions or elixirs are desired for oral administration, the essential active ingredient therein may be combined with various sweetening or flavoring agents, coloring matter or dyes and, if desired, emulsifying or suspending agents, together with diluents such as water, ethanol, propylene glycol, glycerin and combinations thereof.

For parenteral administration, solutions containing an active compound of this invention or a pharmaceutically acceptable salt thereof in sesame or peanut oil, aqueous propylene glycol, or in sterile aqueous solution may be employed. Such aqueous solutions should be suitably buffered if necessary and the liquid diluent first rendered isotonic with sufficient saline or glucose. These particular aqueous solutions are especially suitable for intravenous, intramuscular, subcutaneous and intraperitoneal administration. The sterile aqueous media employed are all readily available by standard techniques known to those skilled in the art.

The effective dosages for compounds of the formula I and their salts will depend on the intended route of administration and factors such as the age and weight of the patient, as generally known to a physician. The dosages will also depend on the particular illness to be treated. For instance, the daily dosage for stress-induced illnesses, inflammatory disorders, Alzheimer's disease, gastro-intestinal diseases, anorexia nervosa, hemorrhagic stress and drug and alcohol withdrawal symptoms will generally range from about 0.1 to about 50 mg/kg body weight of the patient to be treated.

Methods that may be used to determine the CRF antagonist activity of the active compounds of this invention and their pharmaceutically acceptable salts are described in Endocrinology, 116, 1653-1659 (1985) and Peptides, 10, 179-188 (1985). The binding activities for compounds of formulas I, II and III, expressed as IC₅₀ values, generally range from about 0.5 nanomolar to about 10 micromolar.

The present invention is illustrated by the following examples. It will be understood, however, that the invention is not limited to the specific details of these examples. Melting points are uncorrected. Proton nuclear magnetic resonance spectra (¹H NMR) and C¹³ nuclear magnetic resonance spectra (C¹³ NMR) were measured for solutions in deuterochloroform (CDCl₃) and peak positions are expressed in parts per million (ppm) downfield from tetramethylsilane (TMS). The peak shapes are denoted as follows: s, singlet; d, doublet; t, triplet; q, quartet; m, multiplet; b, broad.

The following abbreviations are used in the Examples: Ph=phenyl; iPr=isopropyl; HRMS=high resolution mass spectrum.

### EXAMPLE 1

### 2,5,6-Trimethyl7-(1-propylbutyl)-4-(2,4,6-trimethylphenoxy)-7H-pyrrolo[2,3-d]pyrimidine

To a solution of 2,4,6-trimethylphenol (111 mg, 0.82 mmol) in 3 ml of DMSO was added 60% sodium hydride (NaH) in oil (32 mg, 0.8 mmol). After stirring for 10 min, 4-chloro-2,5,6-trimethyl-7-(1-propylbutyl)-7H-pyrrolo[2,3,4]pyrimidine (200 mg, 0.68 mmol) was added. The resulting mixture was heated at 135°C in an oil bath for 3 hours. An additional 10 mg of 60% NaH was added and the mixture was heated at 135°C for an additional 1 hour and cooled to room temperature. The mixture was quenched with water and extracted with ethyl acetate (EtOAc). The organic layer was washed with 2N sodium hydroxide (NaOH) and brine, and then dried and concentrated to give a brown oil. The oil was purified through silica gel column chromatography using chloroform (CHCl₃):hexane=4:1 as eluent to give the title compound (79%) as a light green oil. ¹H NMR (CDCl₃) δ 6.92 (s, 2H), 2.43 (s, 3H), 2.42 (s, 3H), 2.33 (s, 6H), 2.12 (s, 6H), 1.7-1.9 (m, 3H), 0.95-1.35 (m, 6H), 0.88 (s, 6H) ppm. MS : [P+] = 393 (100%). The corresponding HCI salt was also prepared.

### EXAMPLE 2

### 1-(1-Ethylpropyl)-6-methyl-4-(2,4,6-trimethylphenylamino)-1,3-dihydroimidazo[4,5-c]pyridin-2-one

To a solution of N4-(1-ethylpropyl)-6-methyl-N2-(2,4,6-trimethylphenyl)-pyridine-2,3,4-triamine (250 mg, 0.77 mmol) in 5 ml of dry tetrahydrofuran (THF) was treated with triphosgene (89 mg, 0.3 mmol) and triethylamine (189 mg, 1.87 mmol) at 0°C and stirred at room temperature for 0.5 hours. The mixture was quenched with water and extracted with ethyl acetate. The organic layer was dried and concentrated to give 260 mg of a tan solid. The residue was purified through silica gel column chromatography to give 200 mg of the title compound (> 90% pure) and 60 mg of white crystals of the title compound. Mp 148-150°C. ¹H NMR (CDCl₃) δ 6.96 (s, 2H), 6.39 (s, 1H), 6.00 (s, 1H, NH), 5.94 (s, 1H, NH), 4.03 (m, 1H), 2.44 (s, 3H), 2.32 (s, 3H), 2.20 (s, 6H), 1.80-2.05 (m, 4H), 0.82 (t, 6H) ppm.

The following compounds were prepared by a method analogous to that described in Example 2 starting from the appropriate 4-substituted-N-(1-ethyl-propyl)-2-methyl-pyrimidine-5,6-diamineor2-substituted-N4-(1-ethylpropyl)-6-methyl-pyridine-3,4-diamine and purified from silica gel column chromatography.

### EXAMPLE 3

### 9-(1-Ethylpropyl)-2-methyl-6-(2,4,6-trimethylphenylamino)-7,9-dihydro-purin-8-one

¹H NMR (CDCl₃) δ 6.98 (s, 2H), 6.81 (s, 1H), 5.709 (brs, 1H), 4.14 (m, 1H), 2.44 (s, 3H), 2.33 (s, 3H), 2.20 (s, 6H), 2.0-2.3 (m, 2H), 1.8-2.0 (s, 3H), 0.81 (t, 6H) ppm.

### EXAMPLE 4

### 1-(1-Ethylpropyl)-6-methyl-4-(2,4,6-trimethylphenoxy)-1,3-dihydroimidazo[4,5-c]pyridin-2-one

Mp 235-237°C. Anal. calc'd for C₂₁H₂₇N₃O₂ (C,H,N) **[Fill In date or Delete].** ¹H NMR (CDCl₃) δ 7.02 (s, 1H), 6.91 (s, 2H), 6.61 (s, 1H), 4.12 (m, 1H), 2.39 (s, 3H), 2.32 (s, 3H), 2.12 (s, 6H), 1.8-2.1 (m, 4H), 0.87 (t, 6H) ppm.

### EXAMPLE 5

### 1-(1-Ethylpropyl)-6-methyl-4-(2,4,6-trimethylphenoxy)-1H-imidazo[4,5-c]pyridine

A mixture of N4-(1-ethylpropyl)-6-methyl-2-(2,4,6-trimethylphenoxy)-pyridine-3,4-diamine (160 mg, 0.49 mmol), trimethyl orthoformate (62 mg, 0.59 mmol) and para-tosylalcohcl (p-TsOH) (10 mg) in 20 ml of toluene was heated at reflux under a Dean-Stark trap apparatus for 24 hours. The mixture was quenched with water and extracted with ethyl acetate. The organic layer was dried and concentrated to give the title compound (160 mg, 97%) as a light brown oil. The oil was purified through silica gel column chromatography using 2% methanol (MeOH) in chloroform as eluent to give a tan solid. Mp 127-131°C. ¹H NMR (CDCl₃) δ 7.82 (s, 1H), 6.90 (s, 2H, 6.81) (s, 1H), 4.02 (m, 1H), 2.37 (s, 3H), 2.32 (s, 3H), 2.13 (s, 6H), 1.98 (m, 4H), 0.87 (t, 6H) ppm.

### EXAMPLE 6

### 1-(1-Ethylpropyl)-3,6-dimethyl-4-(2,4,6-trimethylphenoxy)-1,3-dihydroimidazo[4,5-c]pyridin-2-one

A solution of 1-(1-ethylpropyl)-6-methyl-4-(2,4,6-trimethylphenoxy)-1,3-dihydroimidazo[4,5-c]pyridin-2-one (100 mg, 0.28 mmol) in 5 ml of dry THF was treated with lithium bis(trimethylsilyl)amide (0.31 ml, 1M in THF, 0.31 mmol) at -78°C. After 20 min, the mixture was quenched with 1 ml of methyl iodide and stirred at room temperature for 1 hour. The mixture was quenched with water and extracted with ethyl acetate. The organic layer was dried and concentrated to give 110 mg of an off-white solid which was recrystallized from isopropyl ether to give white crystals. Mp 152-154°C; ¹H NMR (CDCl₃) δ 6.91 (s, 2H), 6.57 (s, 1H), 4.18 (m, 1H), 3.73 (s, 3H), 2.32 (s, 3H), 2.27 (s, 3H), 2.12 (s, 6H), 1.9-2.1 (m, 2H), 1.7-1.9 (m, 2H), 0.88 (t, 6H) ppm.

### EXAMPLE 7

### 1-(1-Ethypropyl)-3,6-dimethyl-4-(2,4,6-trimethylphenylamino)-1,3-dihydroimidazo[4,5-c]pyridin-2-one

The title compound was prepared by a method analogous to that described in Example 6 starting from 1-(1-ethylpropyl)-6-methyl-4-(2,4,6-trimethylphenylamino)-1,3-dihydro-imidazo[4,5-c]pyridin-2-one. ¹H NMR (CDCl₃) δ 6.91 (s, 2H), 6.42 (s, 1H), 5.77 (s, 1H), 4.13 (m, 1H), 3.49 (s, 3H), 2.31 (s, 6H), 2.17 (s, 6H), 1.9-2.2 (m, 2H), 1.7-1.9 (m, 2H), 0.86 (t, 6H) ppm.

### EXAMPLE 8

### 1-(1-Ethyl-propyl)-6-methyl-4-(2,4,6-trimethyl-phenoxy)-1H-[1,2,3]triazolo[4,5-c]pyridine

To a solution of 2-(2,4,6-trimethylphenoxy)-N4-(1-ethylpropyl)-6-methyl-pyridine-3,4-diamine (640 mg, 1.95 mmol) and 7 ml of 48% hydrobromic acid was added a solution of sodium nitrite (146 mg, 2.11 mmol) in 2 ml of water dropwise over 5 min at 0°C. The resulting mixture was treated with cuprous bromide Cu(l)Br (145 mg, 1.01 mmol) and then heated at reflux for 15 min. The mixture was cooled to room temperature and diluted with water, basified with ammonium hydroxide and extracted twice with ethyl acetate. The organic layer was dried and concentred to give 710 mg (93% yield) of the title compound as brown crystals, which was further recrystallized from isopropyl ether to give the title compound as golden crystals. ¹H NMR (CDCl₃) δ 6.92(s,2H), 6.84(s,1H), 4.5(m,1H), 2.40(s,3H), 2.32(s,3H), 2.13(s,6H), 2.0-2.4(m,4HO, 0.83(t,6H)ppm.

### EXAMPLE 9

### 7-Bromo-1-(1-ethyl-propyl)-6-methyl-4-(2,4,6-trimethyl-phenoxy)-1H-[1,2,3] triazolo[4,5-c]pyridine

A mixture of 2-(2,4,6-trimethylphenoxy)-N4-(1-ethylpropyl)-6-methyl-pyridine-3,4-diamine (250 mg, 0.763 mmol), n-butyl nitrite (118 mg, 1.15 mmol) and CuBr₂ (205 mg, 0.916 mmol) in anhydrous acetonitrile was heated at 65°C for 2 hours. The mixture was quenched with 16 ml of 2N HCI and extracted 3 times with ethyl acetate. The organic layer was dried and concentrated to give a light brown form (0.310 g). The crude material was purified through silica gel column chromatography using 1:1 chloroform:ethyl acetate as eluent to give 160 mg of 1-(1-ethyl-propyl)-6-methyl-4-(2,4,6-trimethyl-phenoxy)-1H-[1,2,3]triazolo [4,5-c]pyridine and 60 mg of 7-bromo-1-(1-ethyl-propyl)-6-methyl-4-(2,4,6- trimethyl-phenoxy)-1H-[1,2,3]triazolo [4,5-c]pyridine. Mp 154-156°C; ¹H NMR (CDCl₃) δ 6.92(s,2H), 5.5(m,1H), 2.51(s,3H), 2.33(s,3H), 2.13(s,6H), 2.2-2.45(m,2H), 2.0-2.2 (m,2H), 0.87(t,6H) ppm.

### EXAMPLE 10

### 1-(1-Ethyl-propyl)-6,7-dimethyl-4-(2,4,6-trimethyl-phenoxy)-1H-[1,2,3] triazolo[4,5-c]pyridine

To a -78°C solution of 7-bromo-1-(1-ethyl-propyl)-6-methyl-4-(2,4,6-trimethyl-phenoxy)-1H-[1,2,3]triazolo[4,5-c]pyridine (33 mg, 0.079 mmol) in 2 ml of dry THF was added 2.5 M nBuLi in hexane (0.047 ml, 0.019 mmol) and stirred at that temperature for 5 min. An excess of Mel (0.5 ml) was added and the mixture was stirred at that temperature for 15 min, then gradually warmed to room temperature for 1 hour. The mixture was quenched with saturated ammonium chloride and extracted with ethyl acetate. The organic layer was dried and concentrated to give 31 mg of a golden oil. The oil was purified through silica gel column chromatography using 5% ethyl acetate in hexane as eluent to give the title compound as white crystals. Mp 127.129°C; ¹H NMR (CDCl₃) δ 6.91 (s,2H), 4.83(m,1H), 2.51(s,3H), 2.38(s,3H), 2.33(s,3H), 2.13(s,6H), 2.3-2.5(m,2H), 1.9-2.2(m,2H), 0.86(t,6H) ppm.

### EXAMPLE 11

### 1-(1-Ethyl-propyl)-6-methyl-4-(2,4,6-trimethyl-phenoxy)-1,3-dihydro-pyrrolo [3,2-c]pyridin-2-one

A mixture of [4-(1-ethyl-propylamino)-6-methyl-2-(2,4,6-trimethyl-phenoxy)-pyridin-3-yl]-acetonitrile (800 mg, 2.27 mmol) , 6 ml of 85% phosphoric acid and 2 ml of water was heated at reflux for 2 hours and cooled to room temperature. The reaction mixture was neutralized with 2N NaOH and extracted twice with chloroform. The chloroform layer was dried and concentrated to give a yellow solid. The solid was purified through silica gel column chromatography using hexane to 6% ethyl acetate in hexane as eluent to give 730 mg (92.2%) of a white solid. ¹H NMR (CDCl₃) δ 6.87(s,2H), 6.5(s,1H), 4.1(m,1H), 3.12(s,2H), 2.38(s,3H), 2.30(s,3H), 2.10(s,3H), 1.7-2.0(m,4H), 0.8(t,6H) ppm.

### EXAMPLE 12

### 1-(1-Ethyl-propyl)-6-methyl-4-(2,4,6-trimethyl-phenoxy)-1H-pyrrolo[3,2-c]pyridine

A mixture of 1-(1-Ethyl-propyl)-6-methyl-4-(2,4,6-trimethyl-phenoxy)-1,3-dihydro-pyrrolo[3,2-c]pyridin-2-one (12 mg, 0.034 mmol) and 2M BH3-DMS complex in THF (0.1 ml, 0.2 mmol) in 1 ml of dry THF was heated at reflux for 3 hours. The mixture was quenched with dilute HCI and stirred for 1 hour, then neutralized, and extracted with ethyl acetate. The organic layer was dried and concentrated. The residue was purified through silica gel column chromatography using hexane to 4% ethyl acetate in hexane as eluent to give 6 mg of the title compound. ¹H NMR (CDCl₃) δ 6.88(s,2H), 6.84(s,1H), 6.74(s,1H), 5.97(s,1H), 4.00(m,1H), 2.43(s,3H), 2.30(s,3H), 2.10(s,6H), 1.7-1.9(m,4H), 0.75(t,6H) ppm.

### EXAMPLE 13

### 1-(1-Ethyl-propyl)-6-methyl-4-(2,4,6-trimethyl-phenoxy)-2,3-dihydro-1H-pyrrolo[3,2-c]pyridine

A mixture of 1-(1-Ethyl-propyl)-6-methyl-4-(2,4,6-trimethyl-phenoxy)-1,3-dihydro-pyrrolo[3,2-c]pyridin-2-one (49 mg, 0.142 mmol) and 2M BH3-DMS complex in THF (0.5 ml, 1.0 mmol) in 1 ml of dry THF was heated at reflux for 3 hours. The mixture was quenched with dilute HCl and stirred for 48 hours, then neutralized, and extracted with ethyl acetate. The organic layer was dried and concentrated. The residue was purified through silica gel column chromatography using hexane to 20% ethyl acetate in hexane as eluent to give 15 mg (31%) of the title compound as a clear oil and 18 mg (38%) of 1-(1-Ethyl-propyl)-6-methyl-4-(2,4,6-trimethyl-phenoxy)-1H-pyrrolo[3,2-c]pyridine. ¹H NMR (CDCl₃) of the title compound: δ 6.84(s,2H), 5.89(s,1H), 3.3(t,2H), 3.2(m,1H), 2.5(t,2H), 2.28(s,6H), 2.14(s,6H), 1.4-1.6(m,4H), 0.88(t,6H)ppm.

### EXAMPLE 14

### 1-(1-Ethyl-propyl)-6-methyl-4-(2,4,6-trimethyl-phenoxy)-1H-imidazo[4,5-c]pyridin-2-ylamine

A mixture of of 2-(2,4,6-trimethylphenoxy)-N4-(1-ethylpropyl)-6-methylpyridine-3,4-diamine (200 mg, 0.611 mmol) and 5M BrCN in acetonitrile (0.12 ml, 0.611 mmol) in 3 ml of anhydrous acetonitrile was stirred at room temperature overnight. The mixture was quenched with water and saturated sodium bicarbonate and extracted 3 times with ethyl acetate. The organic extracts was washed with brine, dried and concentrated to give 240 mg of a light green form. The residue was purified through silica gel column chromatography using 10% methanol in chloroform as eluent to give 146 mg (68%) of the title compound as a tan solid. Mp 208-210°C. ¹H NMR (CDCl₃) δ 6.89 (s,2H), 6.68(s,1H), 5.03(s,2H), 3.84(m,1H), 2.31(s,6H), 2.13(s,6H), 1.8-2.2(m,4H), 0.89(t,6H) ppm.

### EXAMPLE 15

### 1-(1-Ethyl-propyl)-3,6-dimethyl-4-(2,4,6-trimethyl-phenoxy)-1,3-dihydro-pyrrolo[3,2-c]pyridin-2-one

To a -78°C solution of 1-(1-ethyl-propyl)-6-methyl-4-(2,4,6-trimethylphenoxy)-1,3-dihydro-pyrrolo[3,2-c]pyridin-2-one (352 mg, 1.0 mmol) in 2 ml of dry THF was added 2.5M BuLi in hexane (0.4 mmol, 1.0 mmol). The resulting mixture was stirred at -78°C for 30 min, then transferred to a -78°C solution of methyl iodide (3 ml) in 3ml of dry THF. The resulting mixture was stirred at -78°C for 1 hour, quenched with saturated ammonium chloride, extracted with ethyl acetate. The organic layer was dried and concentrated to give a clear oil which was purified through silica gel column chromatography using hexane to 10% ethyl acetate in hexane as eluent to give the title compound as tan solid 214 mg (68%). ¹H NMR (CDCl₃) δ 6.88 (s,2H), 6.47(s,1H), 4.1(m,1H), 3.56(q,1H), 2.30(s,3H), 2.26(s,3H), 2.07(s,6H), 1.7-2.0(m,4H), 1.60(d,3H), 0.86(t,6H) ppm.

### EXAMPLE 16

### 1-(1-Ethyl-propyl)-3,3,6-trimethyl-4-(2,4,6-trimethyl-phenoxy)-1,3-dihydro-pyrrolo[3,2-c]pyridin-2-one

The title compound was prepared by the method analogous to that described in the Example 15 starting from 1 equivalent of 1-(1-ethyl-propyl)-6-methyl-4-(2,4,6-trimethyl-phenoxy)-1,3-dihydro-pyrrolo[3,2-c]pyridin-2-one and 2.5 equivalent of n-BuLi at -78°C, followed by quenching with excess of methyl iodide. ¹H NMR (CDCl₃) δ 6.88(s,2H), 6.46(s,1H), 4.11(m,1H), 2.29(s,3H), 2.24(s,3H), 2.05(s,6H), 1.8-2.0(m,2H), 1.6-1.8(m,2H), 1.52(s,6H), 0.85(t,6H) ppm.

### EXAMPLE 17

### 1-(1-Ethyl-propyl)-3,3,6-trimethyl-4-(2,4,6-trimethyl-phenoxy)-2,3-dihydro-1H-pyrrolo[3,2-c]pyridine

To a solution of 1-(1-ethyl-propyl)-3,3,6-trimethyl-4-(2,4,6-trimethylphenoxy)-1,3-dihydro-pyrrolo[3,2-c]pyridin-2-one (50 mg) in 2 ml of dry THF was added excess of 2M borane-dimethyl sulfide complex in THF. The resulting mixture was heated at reflux for 6 hours. The mixture was quenched with dilute HCl and stirred for 30 min, neutralized with 2N NaOH, brine and extracted with ethyl acetate. The organic layer was dried and concentrated to givethe solid. The solid was purified through silica gel column chromatography using 10% ethyl acetate in chloroform as eluent to give the title compound as a white solid. ¹H NMR (CDCl₃) δ 6.86(s,2H), 5.88(s,1H), 3.3(m,1H), 3.2(s,2H), 2.29(s,3H), 2.13(s,3H), 2.09(s,6H), 1.6(m,4H), 1.47(s,6H), 0.91(t,6H) ppm.

### EXAMPLE 18

### 1-(1-Ethyl-propyl)-3,6-dimethyl-4-(2,4,6-trimethyl-phenoxy)-1H-pyrrolo [3,2-c]pyridine

A mixture of 1-(1-ethyl-propyl)-3,6-dimethyl-4-(2,4,6-trimethyl-phenoxy)-1,3-dihydro-pyrrolo[3,2-c]pyridin-2-one (20 mg, 0.0546 mmol) and 2M borane-dimethyl sulfide complex in THF (0.07 ml) in 1 ml of THF was heated at reflux for 2 hours. The mixture was quenched with dilute HCl and stirred for 30 min, then neutralized and extracted with ethyl acetate. The organic layer was dried and concentrated to give the crude residue. The residue was purified through silic gel column chromatography using hexane to 10% ethyl acetate in hexane as eluent to give the title compound as a white solid. ¹H NMR (CDCl₃) δ 6.89(s,2H), 6.69(s,1H), 6.63(s,1H), 3.92(m,1H), 2.49(s,3H), 2.30(s,3H), 2.11(s,6H), 1.7-1.9(m,4H), 0.78(t,6H)ppm.

### EXAMPLE 19

### 1-(1-Ethyl-propyl)-2 -methoxy-3,6-dimethyl-4-(2,4,6-trimethylphenoxy)-1H-pyrrolo[3,2-c]pyridine

To a 0°C solution of 1-(1-Ethyl-propyl)-6-methyl-4-(2,4,6-trimethyl-phenoxy)-1,3-dihydro-pyrrolo[3,2-c]pyridin-2-one (134 mg, 0.381 mmol) in 2ml of HMPA was added 60% sodium hydride in oil (20 mg, 0.5 mmol) and the resulting mixture was stirred at 0°C for 10 min. Dimethyl sulfate (66.5 mg, 0.53 mmol) was added and stirred for 30 min. The reaction mixture was quenched with dilute acid to pH4 and extracted with ethyl acetate. The organic layer was washed with brine, dried and concentrated to give a clear oil. The oil was purified through silica gel column chromatography using 3 % ethyl acetate in hexane as eluent to give 70 mg of the title compound as white solid. ¹H NMR (CDCl₃) δ 6.88(s,2H), 6.61(s,1H), 4.0(m,1H), 3.95(s,3H), 2.44(s,3H), 2.29(s,3H), 2.26(s,3H), 2.10(s,6H), 1.95-2.1(m,2H), 1.7-1.9(m,2H), 0.78(t,6H)ppm.

### EXAMPLE 20

### [1-(1-Ethyl-propyl)-6-methyl-1H-[1,2,3]triazolo[4,5-c]pyridin-4-yl]-(2,4,6-trimethyl-phenyl)-amine

A mixture of N4-(1-ethyl-propyl)-6-methyl-N2-(2,4,6-trimethyl-phenyl)-pyridine-2,3,4-triamine (250 mg, 0.766 mmol) and butyl nitrite (119 mg, 1.15 mmol) in 16 ml of acetonitrile was heated at 65°C for 2 hours. The mixture was quenched with 2N HCl, then neutralized to pH 7 and extracted with ethyl acetate. The organic layer was washed with brine, dried and concentrated to give 250 mg of a golden brown residue. tlc indicated two components were obtained from this reaction, in which the more polar one is the title compound. The title compound was isolated as a white crystals, mp 140-142°C, after silica gel column chromatography using 10% ethyl acetate in hexane as eluent. ¹H NMR (CDCl³) δ 6.94(s,2H), 6.49(s,1H), 4.40(m,1H), 2.38(s,3H), 2.31(s,3H), 2.23(s,6H), 2.05-2.2(m,2H), 1.9-2.05(m,2H), 0.80(t,6H) ppm.

### EXAMPLE 21

### 4-(4-Bromo-2,6-dimethyl-phenoxy)-1-(1-ethyl-propyl)-6-methyl-1H-oxazolo [5,4-c]pyridin-2-one

To a 0°C solution of 4-(1-ethyl-propylamino)-6-methyl-2-(4-bromo-2,6-dimethyl-phenoxy)-pyridin-3-ol (40 mg, 0.101 mmol) was added triphosgene (10 mg, 0.035 mmol) and triethylamine (7 mg, 0.07 mmol) in 1 ml of dry THF. The resulting mixture was stirred overnight. The mixture was quenched with water and extracted with ethyl acetate. The organic layer was washed with brine, dried and concentrated. The residue was purified through silica gel column chromatography to give 26 mg (61%) of the title compound as a white solid. ¹H NMR (CDCl₃) δ 7.22(s,2H), 6.60(s,1H), 4.02(m,1H), 2.31(s,3H), 2.12(s,6H), 1.8-2.2(m,4H), 0.94(t,6H)ppm.

### EXAMPLE 22

### 1-(1-Ethyl-propyl)-6-methyl-4-(2,4,6-trimethyl-phenoxy)-1H-oxazolo[5,4-c]pyridin-2-one

The title compound was prepared as a grey solid by the method analogous to that described in the Example 21 starting from 4-(1-Ethyl-propylamino)-6-methyl-2-(2,4,6-trimethyl-phenoxy)-pyridin-3-ol and triphosgene. ¹H NMR (CDCl₃) δ 6.87(s,2H), 6.55(s,1H), 3.98(m,1H), 2.29(s,3H), 2.28(s,3H), 2.09(s,6H), 1.9-2.05(m,2H), 1.8-1.9(m,2H), 0.90(t,6H) ppm.

### EXAMPLES 23(a) - 23(g)

The following compounds can be prepared by the method analogous to that described in Example 11 starting from [4-(1-ethyl-propylamino)-5-methyl-2-(substituted-phenoxy)-pyridin-3-yl]-acetonitrile and phosphoric acid.
(a) **1-(1-Ethyl-propyl)-6-methyl-4-(4-chloro-2,6-dimethyl-phenoxy)-1,3-dihydro-pyrrolo[3,2-c]pyridin-2-one:**
(b) **1-(1-Ethyl-propyl)-6-methyl-4-(4-bromo-2,6-dimethyl-phenoxy)-1,3-dihydro-pyrrolo[3,2-c]pyridin-2-one:**
(c) **1-(1-Ethyl-propyl)-6-methyl-4-(2-bromo-4-i-propylphenoxy)-1,3-dihydro-pyrrolo[3,2-c]pyridin-2 -one:**
(d) **1-(1-Ethyl-propyl)-6-methyl-4-(2,4-dimethyl-phenoxy)-1,3-dihydropyrrolo[3,2-c]pyridin-2-one;**
(e) **1-(1-Ethyl-propyl)-6-methyl-4-(4-i-propyl-2,6-dimethyl-phenoxy)-1,3-dihydro-pyrrolo[3,2-c]pyridin-2-one;**
(f) **1-(1-Ethyl-propyl)-6-methyl-4-(4-t-butyl-2,6-dimethyl-phenoxy)-1,3-dyhdro-pyrrolo[3,2-c]pyridin-2-one; and**
(g) **1-(1-Ethyl-propyl)-6-methyl-4-(4-trifluoromethyl-2,6-dimethylphenoxy)-1,3-dihydro-pyrrolo[3,2-c]pyridin-2-one.**

### EXAMPLES 24(a) - 24(i)

The following compounds can be prepared by the method analogous to that described in Example 15 starting from 1-(1-Ethyl-propyl)-6-methyl-4-(substituted-phenoxy)-1,3-dihydro-pyrrolo[3,2-c]pyridin-2-one and an appropriate base, such as BuLi, lithium diisopropylamide, or lithium bis(trimethylsilyl)amide, followed by quenching with an appropriate electrophile such as methyl iodide or ethyl iodide.
(a) **1-(1-Ethyl-propyl)-3,6-dimethyl-4-(4-chloro-2,6-dimethyl-phenoxy)-1,3-dihydro-pyrrolo[3,2-c]pyridin-2-one:**
(b) **1-(1-Ethyl-propyl)-3,6-dimethyl-4-(4-bromo-2,6-dimethylphenoxy)-1,3-dihydro-pyrrolo[3,2-c]pyridin-2-one;**
(c) **1-(1-Ethyl-propyl)-3,6-dimethyl-4-(2-bromo-4-i-propyl-phenoxy)-1,3-dihydro-pyrrolo[3,2-c]pyridin-2-one;**
(d) **1-(1-Ethyl-propyl)-3-ethyl-6-methyl-4-(4-chloro-2,6-dimethylphenoxy)-1,3-dihydro-pyrrolo[3,2-c]pyridin-2-one;**
(e) **1-(Ethyl-propyl)-3-ethyl-6-methyl-4-(4-bromo-2,6-dimethyl-phenoxy)-1,3-dihydro-pyrrolo[3,2-c]pyridin-2-one;**
(f) **1-(1-Ethyl-propyl)-3-ethyl-6-methyl-4-(2-bromo-4-i-propyl-phenoxy)-1,3-dihydro-pyrrolo[3,2-c]pyridin-2-one;**
(g) **1-(1-Ethyl-propyl)-3,6-dimethyl-4-(2,4-dimethyl-phenoxy)-1,3-dihydro-pyrrolo[3,2-c]pyridin-2-one;**
(h) **1-(1-Ethyl-propyl)-3,6-dimethyl-4-(4-i-propyl-2,6-dimethyl-phenoxy)-1,3-dihydro-pyrrolo[3,2-c]pyridin-2-one;**
(i) **1-(1-Ethyl-propyl)-3,6-dimethyl-4-(4-t-butyl-2,6-dimethyl-phenoxy)-1.3-dihydro-pyrrolo[3,2-c]pyridin-2-one;** and
(j) **1-(1-Ethyl-propyl)-3,6-dimethyl-4-(4-trifluoromethyl-2,6-dimethylphenoxy)-1,3-dihydro-pyrrolo[3,2-c]pyridin-2-one.**

### EXAMPLES 25(a) - 25(k)

The following compounds can be prepared by the method analogous to that described in Example 18 starting from 1-(1-Ethyl-propyl)-3,6-dimethyl-4-(substituted-phenoxy)-1,3-dihydro-pyrrolo-[3,2-c]pyridin-2-one.
(a) **1-(1-Ethyl-propyl)-3,6-dimethyl-4-(4-chloro-2.6-dimethylphenoxy)-1H-pyrrolo[3,2-c]pyridine:**
(b) **1-(1-Ethyl-propyl)-3,6-dimethyl-4-(4-bromo-2,6-dimethylphenoxy)-1H-pyrrolo[3,2-c]pyridine;**
(c) **1-(1-Ethyl-propyl)-3,6-dimethyl-4-(2-bromo-4-i-propyl-phenoxy)-1H-pyrrolo[3,2-c]pyridine:**
(d) **1-(1-Ethyl-propyl)-3-ethyl-6-methyl-4-(4-chloro-2,6-dimethylphenoxy)-1H-pyrrolo[3,2-c]pyridine:**
(e) **1-(1-Ethyl-propyl)-3-ethyl-6-methyl-4-(4-bromo-2,6-dimethylphenoxy)-1H-pyrrolo[3,2-c]pyridine;**
(f) **1-(1-Ethyl-propyl)-3-ethyl-6-methyl-4-(2-bromo-4-i-propyl-phenoxy)-1H-pyrrolo[3,2-c]pyridine;**
(g) **1-(1-Ethyl-propyl)-3,6-dimethyl-4-(2-bromo-4-i-propyl-phenoxy)-1H-pyrrolo[3,2-c]pyridine;**
(h) **1-(1-Ethyl-propyl)-3,6-dimethyl-4-(2-bromo-4-i-propyl-phenoxy)-1H-pyrrolo[3,2-c]pyridine;**
(i) **1-(1-Ethyl-propyl)-3,6-dimethyl-4-(4-i-propyl-2,6-dimethyl-phenoxy)-1H-pyrrolo[3,2-c]pyridine:**
(j) **1-(1-Ethyl-propyl)-3,6-dimethyl-4-(4-t-butyl-2,6-dimethyl-phenoxy)-1H-pyrrolo[3,2-c]pyridine; and**
(k) **1-(1-Ethyl-propyl)-3,6-dimethyl-4-(4-trifluoromethyl-2,6-dimethyl-phenoxy)-1H-pyrrolo[3,2-c]pyridine.**

### EXAMPLES 26(a) - 26(g)

### (a) 1-(1-Ethyl-propyl)-3,6-dimethyl-4-(4-ethyl-2,6-dimethyl-phenoxy)-1H-pyrrolo[3,2-c]pyridine

To a solution of 2.5 N n-BuLi in hexane in dry THF was added a solution of leq. of 1-(1-ethyl-propyl)-3,6-dimethyl-4-(4-bromo-2,6-dimethyl-phenoxy)-1H-pyrrolo[3,2-c]pyridine in dry THF at -78°C. After stirring at that temperature for 5 min, an appropriate electrophile (e.g., DMF, formaldehyde, or a C₃-C₄ iodide) was added and the resulting mixture was stirred at -78°C for 30 min, then at 0°C for 15 min. The mixture was quenched with saturated ammonium chloride and extracted with ethyl acetate. The organic layer was dried and concentrated to give the title compound after silica gel column chromatography.

The following compounds can also be prepared using the foregoing procedure:
(b) **1-(1-Ethyl-propyl)-3,6-dimethyl-4-(4-propyl-2,6-dimethyl-phenoxy)-1H-pyrrolo[3,2-c]pyridine;**
(c) **1-(1-Ethyl-propyl)-3,6-dimethyl-4-(4-hydroxymethyl-2,6-dimethyl-phenoxy)-1H-pyrrolo[3,2-c]pyridine;**
(d) **1-(1-Ethyl-propyl)-3,6-dimethyl-4-(4-formyl-2,6-dimethyl-phenoxy)-1H-pyrrolo[3,2-c]pyridine;**
(e) **1-(1-Ethyl-propyl)-3-ethyl-6-methyl-4-(4-propyl-2,6-dimethyl-phenoxy)-1H-pyrrolo[3,2-c]pyridine:**
(f) **1-(1-Ethyl-propyl)-3-ethyl-6-methyl-4-(4-hydroxymethyl-2,6-dimethyl-phenoxy)-1H-pyrrolo[3,2-c]pyridine; and**
(g) **1-(1-Ethyl-propyl)-3-ethyl-6-methyl-4-(4-formyl-2,6-dimethyl-phenoxy)-1H-pyrrolo[3,2-c]pyridine.**

### EXAMPLES 27(a) - 27(f)

The following examples can be prepared by a reaction sequence similar to those described in Examples 11, 15 and 18 (sequentially), starting from [4-(1-hydroxymethylpropylamino)-6-methyl-2-(substituted-phenoxy)-pyridin-3-yl]-acetonitrile.
(a) **2-[4-(4-Chloro-2,6-dimethyl-phenoxy)-3,6-dimethyl-pyrrolo[3,2-c]pyridin-1-yl]-butan-1-ol;**
(b) **2-[4-(4-bromo-2.6-dimethyl-phenoxy)-3,6-dimethyl-pyrrolo[3,2-c]pyridin-1-yl]-butan-1-ol;**
(c) **2-[4-(4-i-propyl-2,6-dimethyl-phenoxy)-3,6-dimethyl-pyrrolo[3,2-c]pyridin-1-yl]-butan-1-ol;**
(d) **2-[4-(4-Ethyl-2,6-dimethyl-phenoxy)-3,6-dimethyl-pyrrolo[3,2-c]pyridin-1-yl-butan-1-ol;**
(e) **2-[4-(4-trifluoromethyl-2,6-dimethyl-phenoxy)-3,6-dimethyl-pyrrolo[3,2-c]pyridin-1-yl]-butan-1-ol;** and
(f) **2-[4-(2-bromo-4-i-propyl-phenoxy)-3,6-dimethyl-pyrrolo[3,2-c]pyridin-1-yl]-butan-ol.**

### PREPARATION A

### 2,5,6-Trimethyl-7-(1-propylbutyl)-7H-pyrrolo[2,3,-d]pyrimidin-4-ol

A mixture of N-[3-cyano-4,5-dimethyl-1-(1-propylbutyl)-lH-pyrrol-2-yl]-acetamide (2.16 g, 7.8 mmol) and 85% phosphoric acid (3.5 ml) was heated at 150°C for 1 hour. The mixture was quenched with water and extracted with chloroform. The organic layer was dried and concentrated to give the title compound as white solid, ¹H NMR (CDCl₃) δ 12.4 (brs, 1H), 4.7 (brs) and 4.0 (brs, total of 1H), 2.46 (s, 3H), 2.36 (s, 3H), 1.6-2.4 (m, 7H), 1.74 (m, 2H), 0.9-1.4 (m, 4H), 0.85 (t, 6H) ppm.

### PREPARATION B

### 4-Chloro-2,5,6-trimethyl-7-(1-propylbutyl)-7H-pyrrolo[2,3,-d]pyrimidine

A mixture of 2,5,6-trimethyl-7-(1-propylbutyl)-7H-pyrrolo[2,3,-d]pyrimidin-4-ol (524 mg, 0.19 mmol) and phosphorous oxychloride (5.5 ml) was heated at reflux overnight. The mixture was cooled and poured into ice and extracted with ethyl acetate. The organic layer was neutralized with sat. sodium carbonate and brine, dried and concentrated to give the title compound as green solid (96%) which was purified through silica gel column chromatography using 1:1 hexane:chloroform as eluent to give the title compound as white crystals. ¹H NMR (CDCl₃) δ 2.68 (s, 3H), 2.38 (s, 6H), 2.32 (brs, 3H), 1.65-1.9 (m, 3H), 0.8-1.35 (m, 6H), 0.84 (t, 6H) ppm.

## Claims

1. A compound of the formula 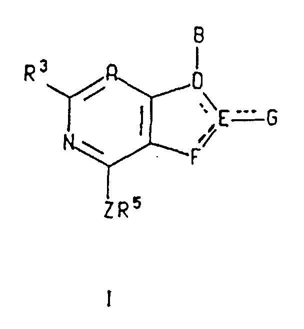 or a pharmaceutically acceptable salt thereof, wherein
the dashed lines represent optional double bonds;
A is nitrogen or CR⁷;
B is -NR¹R², -CR¹R²R¹⁰, -C(=CR²R¹¹)R¹, -NHCR¹R²R¹⁰, -OCR¹R²R¹⁰, -SCR¹R²R¹⁰, -CR²R¹⁰NHR¹, -CR²R¹⁰OR¹, -CR²R¹⁰SR¹ or -COR²;
D is nitrogen and is single bonded to all atoms to which it is attached
E is nitrogen, CH or carbon;
F is oxygen, sulfur, CHR⁴ or NR⁴ when it is single bonded to E and F is nitrogen or CR⁴ when it is double bonded to E;
G, when single bonded to E, is hydrogen, C₁-C₄ alkyl, -S(C₁-C₄ alkyl), -O(C₁-C₄ alkyl), NH₂, -NH(C₁-C₄ alkyl) or -N(C₁-C₂ alkyl)(C₁-C₄ alkyl), wherein each of the C₁-C₄ alkyl groups of G may optionally be substituted with one hydroxy, -O(C₁-C₂ alkyl) or fluoro group; G, when double bonded to E, is oxygen, sulfur or NH; and G, when E is nitrogen and double bonded to D or F, is absent;
R¹ is C₁-C₆ alkyl optionally substituted with one or two substituents R⁸ independently selected from hydroxy, fluoro, chloro, bromo, iodo, C₁-C₄ alkoxy, CF₃, -C(=O)0-(C₁-C₄)alkyl, -OC(=O)(C₁-C₄ alkyl), -OC(=O)N(C₁-C₄ alkyl)(C₁-C₂ alkyl), -NHCO(C₁-C₄ alkyl), -COOH, -COO(C₁-C₄ alkyl), -CONH(C₁-C₄ alkyl), -CON(C₁-C₄ alkyl)(C₁-C₂ alkyl), -S(C₁-C₄ alkyl), -CN, -NO₂, -SO(C₁-C₄ alkyl), -SO₂(C₁-C₄ alkyl), -SO₂NH(C₁-C₄ alkyl) and -SO₂N(C₁-C₄ alkyl)(C₁-C₂ alkyl), wherein each of the C₁-C₄ alkyl groups in the foregoing R¹ groups may optionally contain one or two double or triple bonds;
R² is C₁-C₁₂ alkyl which may optionally contain from one to three double or triple bonds, aryl or (C₁-C₄ alkylene)aryl, wherein said aryl and the aryl moiety of said (C₁-C₄ alkylene)aryl is selected from phenyl, naphthyl, thienyl, benzothienyl, pyridyl, quinolyl, pyrazinyl, pyrimidinyl, imidazolyl, furanyl, benzofuranyl, benzothiazolyl, isothiazolyl, pyrazolyl, pyrrolyl, indolyl, pyrrolopyridyl, oxazolyl and benzoxazolyl; C₃-C₈ cycloalkyl or (C₁-C₆ alkylene)(C₃-C₈ cycloalkyl), wherein one or two of the carbon atoms of said cycloalkyl and the 5 to 8 membered cycloalkyl moieties of said (C₁-C₆ alkylene)(C₃-C₈ cycloalkyl) may optionally and independently be replaced by an oxygen or sulfur atom or by NZ² wherein Z² is selected from hydrogen, C₁-C₄ alkyl, benzyl and C₁-C₄ alkanoyl, and wherein each of the foregoing R² groups may optionally be substituted with from one to three substituents independently selected from chloro, fluoro, hydroxy and C₁-C₄ alkyl, or with one substituent selected from bromo, iodo, C₁-C₆ alkoxy, -OC(=O)(C₁-C₆ alkyl), -OC(=O)N(C₁-C₄ alkyl)(C₁-C₂ alkyl), -S(C₁-C₆ alkyl), amino, -NH(C₁-C₂ alkyl), -N(C₁-C₂ alkyl)(C₁-C₄ alkyl), -N(C₁-C₄ alkyl)-CO-(C₁-C₄ alkyl), -NHCO(C₁-C₄ alkyl), -COOH, -COO(C₁-C₄ alkyl), -CONH(C₁-C₄ alkyl), -CON(C₁-C₄ alkyl)(C₁-C₂ alkyl), -SH, -CN, -NO₂, -SO(C₁-C₄ alkyl), -SO₂(C₁-C₄ alkyl), -SO₂NH(C₁-C₄ alkyl) and -SO₂N(C₁-C₄ alkyl)(C₁-C₂ alkyl);
-NR¹R² may form a saturated 3 to 8 membered carbocyclic ring which may optionally contain from one to three double bonds and wherein one or two of the ring carbon atoms of such 5 to 8 membered rings may optionally and independently be replaced by an oxygen or sulfur atom or by NZ³ wherein Z³ is hydrogen, C₁-C₄ alkyl, benzyl or C₁-C₄ alkanoyl;
R³ is hydrogen, C₁-C₄ alkyl, -O(C₁-C₄ alkyl), chloro, fluoro, bromo, iodo, -CN, -S(C₁-C₄ alkyl) or -SO₂(C₁-C₄ alkyl) wherein each of the (C₁-C₄ alkyl) moieties in the foregoing R³ groups may optionally be substituted with one substituent R⁹ selected from hydroxy, fluoro and (C₁-C₂ alkoxy);
each R⁴ is, independently, hydrogen, (C₁-C₆ alkyl), fluoro, chloro, bromo, iodo, hydroxy, cyano, amino, nitro, -O(C₁-C₄ alkyl), -N(C₁-C₄ alkyl)(C₁-C₂ alkyl), -S(C₁-C₄ alkyl), -SO(C₁-C₄ alkyl), -SO₂(C₁-C₄)alkyl, -CO(C₁-C₄ alkyl), -C(=O)H or -C(=O)O(C₁-C₄alkyl), wherein each of the (C₁-C₆ alkyl) and (C₁-C₄ alkyl) moieties in the foregoing R⁴ groups may optionally contain one or two double or triple bonds and may optionally be substituted with one or two substituents independently selected from hydroxy, amino, C₁-C₃ alkoxy, dimethylamino, methylamino, ethylamino, -NHC(=O)CH₃, fluoro, chloro, C₁-C₃ thioalkyl, -CN, -COOH, -C(=O)O(C₁-C₄ alkyl), -C(=O)(C₁-C₄ alkyl) and -NO₂;
R⁵ is phenyl, naphthyl, thienyl, benzothienyl, pyridyl, quinolyl, pyrazinyl, furanyl, benzofuranyl, benzothiazolyl, benzisothiazolyl, benzisoxazolyl, benzimidazolyl, indolyl, benzoxazolyl or C₃-C₈ cycloalkyl wherein one or two of the carbon atoms of said cycloalkyl rings that contain at least 5 ring members may optionally and independently be replaced by an oxygen or sulfur atom or by NZ⁴ wherein Z⁴ is hydrogen, C₁-C₄ alkyl or benzyl; and wherein each of the foregoing R⁵ groups is substituted with from one to four substituents R¹² wherein one to three of said substituents may be selected, independently, from chloro, C₁-C₆ alkyl and -O(C₁-C₆ alkyl) and one of said substituents may be selected from bromo, iodo, formyl, -CN, -CF₃, -NO₂, -NH₂, -NH(C₁-C₄ alkyl), -N(C₁-C₂ alkyl)(C₁-C₆ alkyl), -C(=O)O(C₁-C₄ alkyl), -C(=O)(C₁-C₄ alkyl), -COOH, -SO₂NH(C₁-C₄ alkyl), -SO₂N(C₁-C₂ alkyl)(C₁-C₄ alkyl), -SO₂NH₂, -NHSO₂(C₁-C₄ alkyl), -S(C₁-C₆ alkyl) and -SO₂(C₁-C₆ alkyl), and wherein each of the C₁-C₄ alkyl and C₁-C₆ alkyl moieties in the foregoing R⁵ groups may optionally be substituted with one or two substituents independently selected from fluoro, hydroxy, amino, methylamino, dimethylamino and acetyl;
R⁷ is hydrogen, C₁-C₄ alkyl, halo, cyano, hydroxy, -O(C₁-C₄ alkyl) -C(=O)(C₁-C₄ alkyl), -C(=O)O(C₁-C₄alkyl), -OCF₃, -CF₃, -CH₂OH, -CH₂O(C₁-C₄ alkyl);
R¹⁰ is hydrogen, hydroxy, methoxy or fluoro;
R¹¹ is hydrogen or C₁-C₄ alkyl; and
Z is NH, oxygen, sulfur, -N(C₁-C₄ alkyl), -NC(=O)(C₁-C₂ alkyl), NC(=O)O(C₁-C₂alkyl) or CR¹³R¹⁴ wherein R¹³ and R¹⁴ are independently selected from hydrogen, trifluoromethyl and methyl with the exception that one of R¹³ and R¹⁴can be cyano;
with the proviso that: (a) in the five membered rings of structure I, there can not be two double bonds adjacent to each other; and (b) when R⁴ is attached to nitrogen, it is not halo, cyano or nitro;
or a pharmaceutically acceptable salt of such compound.

2. A compound according to claim 1 wherein: R¹ is C₁-C₆ alkyl, which may optionally be substituted with one hydroxy, fluoro, CF₃, or C₁-C₄ alkoxy group and may optionally contain one double or triple bond; and R² is benzyl, C₁-C₆ alkyl, which may optionally contain one double or triple bond, wherein said C₁-C₆ alkyl and the phenyl moiety of said benzyl may optionally be substituted with one fluoro, CF₃, C₁-C₂ alkyl, C₁-C₂ alkoxy or chloro group.

3. A compound according to claim 1 wherein: R³ is methyl, ethyl, chloro or methoxy; R⁴ is methyl, ethyl or trifluoromethyl; G is hydrogen, methyl, ethyl, or E=G is C=O, C=S; R⁵ is phenyl, pyridyl, pyrimidyl which is substituted with more than two substituents independently selected from C₁-C₄ alkyl, -O(C₁-C₄ alkyl), (C₁-C₄ alkyl)-O-(C₁-C₄ alkyl), CF₃, OCF₃, -CHO, (C₁-C₄ alkyl)-OH, CN, Cl, F, Br, I and NO₂, wherein each of the foregoing (C₁-C₄) alkyl groups may optionally contain one double or triple bond.

4. A compound according to claim 1 wherein A is N, CH or CCH₃ which may optionally be substituted by fluoro, chloro, CF₃, C₁-C₄ alkyl or C₁-C₄ alkoxy.

5. A compound according to claim 1 wherein F is NR⁴.

6. A compound according to claim 1 wherein F is CHR⁴.

7. A compound according to claim 1 wherein F is nitrogen and is double bonded to E.

8. A compound according to claim 1 wherein F is sulfur.

9. A compound according to claim 1 wherein E is carbon.

10. A compound according to claim 1 wherein E is nitrogen.

11. A compound according to claim 1 wherein E is NR²⁵ and R²⁵ is hydrogen, C₁-C₄ alkyl or -CF₃.

12. A compound according to claim 1 that is selected from:
2,5,6-trimethyl-7-( 1-propylbutyl)-4-(2,4,6-trimethylphenoxy)-7H-pyrrolo[2,3-d]pyrimidine;
1-(1-ethylpropyl)-6-methyl-4-(2,4,6-trimethylphenylamino)-1,3-dihydro-imidazo[4,5-c]pyridin-2-one;
9-(1-ethylpropyl)-2-methyl-6-(2,4,6-trimethylphenylamino)-7,9-dihydro-purin-8-one;
1-(1-ethylpropyl)-6-methyl-4-(2,4,6-trimethylphenoxy)-1,3-dihydro-imidazo[4,5-c]pyridin-2-one;
1-(1-ethylpropyl)-6-methyl-4-(2,4,6-trimethylphenoxy)-1H-imidazo[4,5-c]pyridine;
1-(1-ethylpropyl)-3,6-dimethyl-4-(2,4,6-trimethylphenoxy)-1,3-dihydro-imidazo[4,5-c]pyridin-2-one; and
1-(1-ethylpropyl)-3,6-dimethyl-4-(2,4,6-trimethylphenylamino)-1,3-dihydro-imidazo[4,5-c]pyridin-2-one.

13. A pharmaceutical composition comprising a compound or salt thereof as claimed in any preceding claim, and a pharmaceutically acceptable diluent or carrier.

14. A compound or salt as claimed in any of claims 1 to 12, or a composition thereof as claimed in claim 13, for use as a medicament.

15. The use of a compound or salt as claimed in any of claims 1 to 12, or of a composition as claimed in claim 13, for the manufacture of a medicament for the treatment of (a) a disorder the treatment of which can be effected or facilitated by antagonizing CRF, including but not limited to disorders induced or facilitated by CRF, or (b) a disorder selected from inflammatory disorders such as rheumatoid arthritis and osteoarthritis, pain, asthma, psoriasis and allergies; generalized anxiety disorder; panic; phobias; obsessive-compulsive disorder; post-traumatic stress disorder; hypertension; tachycardia; congestive heart failure; sleep disorders induced by stress; pain perception such as fibromyalgia; mood disorders such as depression, including major depression, single episode depression, recurrent depression, child abuse induced depression, and postpartum depression; dysthemia; bipolar disorders; cyclothymia; fatigue syndrome; stress-induced headache; cancer; irritable bowel syndrome; Crohn's disease; spastic colon; human immunodeficiency virus infections; neurodegenerative diseases such as Alzheimer's disease, Parkinson's disease and Huntington's disease; gastrointestinal disorders; eating disorders such as anorexia and bulimia nervosa; hemorrhagic stress; stress-induced psychotic episodes; euthyroid sick syndrome; syndrome of inappropriate antidiarrhetic hormone; obesity; infertility; head traumas; spinal cord trauma; ischemic neuronal damage; excitotoxic neuronal damage; epilepsy; stroke; ulcers; immune dysfunctions including stress induced immune dysfunctions; muscular spasms; urinary incontinence; senile dementia of the Alzheimer's type; multiinfarct dementia; amyotrophic lateral sclerosis; chemical dependencies and addictions; drug and alcohol withdrawal symptoms; psychosocial dwarfism; and hypoglycemia in a mammal.

## Patentansprüche

1. Verbindung der Formel 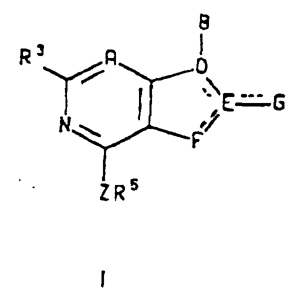 oder ein pharmazeutisch verträgliches Salz davon, worin
die Strichlinien mögliche Doppelbindungen wiedergeben;
A Stickstoff oder CR⁷ darstellt;
B -NR¹R², -CR¹R²R¹⁰, -C(=CR²R¹¹)R¹, -NHCR¹R²R¹⁰, -OCR¹R²R¹⁰, -SCR¹R²R¹⁰, -CR²R¹⁰NHR¹, -CR²R¹⁰OR¹, -CR²R¹⁰SR¹ oder -COR² darstellt;
D Stickstoff darstellt und eine Einfachbindung an alle Atome, an die es gebunden ist, darstellt;
E Stickstoff, CH oder Kohlenstoff darstellt;
F Sauerstoff, Schwefel, CHR⁴ oder NR⁴ darstellt, wenn es an E einfach gebunden ist und F Stickstoff oder CR⁴ darstellt, wenn es an E doppelt gebunden ist;
G, wenn einfach gebunden an E, Wasserstoff, C₁-C₄-Alkyl, -S(C₁-C₄-Alkyl), -O(C₁-C₄-Alkyl), NH₂, -NH(C₁-C₄-Alkyl) oder -N(C₁-C₂-Alkyl)(C₁-C₄-alkyl) darstellt, worin jede der C₁-C₄-Alkylgruppen von G gegebenenfalls mit einer Hydroxy-, -O(C₁-C₂-Alkyl)- oder Fluorgruppe substituiert sein kann; G, wenn doppelt gebunden an E, Sauerstoff, Schwefel oder NH darstellt; und G, wenn E Stickstoff darstellt und doppelt an D oder F gebunden ist, nicht vorliegt;
R¹ C₁-C₆-Alkyl, gegebenenfalls substituiert mit einem oder zwei Substituenten R⁸, unabhängig ausgewählt aus Hydroxy, Fluor, Chlor, Brom, Jod, C₁-C₄-Alkoxy, CF₃, -C(=O)O(C₁-C₄)-Alkyl, -OC(=O) (C₁-C₄-Alkyl), -OC(=O)N(C₁-C₄-Alkyl)(C₁-C₂alkyl), -NHCO(C₁-C₄)-Alkyl, -COOH, -COO(C₁-C₄-Alkyl), -CONH(C₁-C₄-Alkyl), -CON(C₁-C₄-Alkyl)(C₁-C₂-alkyl), -S(C₁-C₄-Alkyl), -CN, -NO₂, -SO(C₁-C₄-Alkyl), -SO₂(C₁-C₄-Alkyl), -SO₂NH(C₁-C₄-Alkyl) und -SO₂N(C₁-C₄-Alkyl)(C₁-C₂-alkyl), worin jede der C₁-C₄-Alkylgruppen in den vorangehenden Gruppen R¹ gegebenenfalls eine oder zwei Doppel- oder Dreifachbindungen enthalten kann, darstellt;
R² C₁-C₁₂-Alkyl, das gegebenenfalls eine bis drei Doppel- oder Dreifachbindungen enthalten kann, Aryl oder (C₁-C₄-Alkylen)aryl, wobei das Aryl und die Aryleinheit des (C₁-C₄-Alkylen)aryl aus Phenyl, Naphthyl, Thienyl, Benzothienyl, Pyridyl, Chinolyl, Pyrazinyl, Pyrimidinyl, Imidazolyl, Furanyl, Benzofuranyl, Benzothiazolyl, Isothiazolyl, Pyrazolyl, Pyrrolyl, Indolyl, Pyrrolopyridyl, Oxazolyl und Benzoxazolyl ausgewählt ist; C₃-C₈-Cycloalkyl oder (C₁-C₆-Alkylen)(C₃-C₈-cycloalkyl), worin ein oder zwei der Kohlenstoffatome des Cycloalkyl und der 5- bis 8-gliedrigen Cyclalkylreste des (C₁-C₆-Alkylen)(C₃-C₈-cycloalkyl) gegebenenfalls und unabhängig durch ein Sauerstoff- oder Schwefelatom oder durch NZ², worin Z² ausgewählt ist aus Wasserstoff, C₁-C₄-Alkyl, Benzyl und C₁-C₄-Alkanoyl, ersetzt sind und worin jede der vorangehenden Gruppen R² gegebenenfalls mit einem bis drei Substituenten, unabhängig ausgewählt aus Chlor, Fluor, Hydroxy und C₁-C₄-Alkyl, oder mit einem Substituenten, ausgewählt aus Brom, Jod, C₁-C₆-Alkoxy, -OC(=O) (C₁-C₆-Alkyl), -OC(=O)N(C₁-C₄-Alkyl) (C₁-C₂-alkyl), -S(C₁-C₆-Alkyl), Amino, -NH(C₁-C₂-Alkyl), -N(C₁-C₂-Alkyl)(C₁-C₄-alkyl), -N(C₁-C₄-Alkyl)-CO-(C₁-C₄-alkyl), -NHCO(C₁-C₄-Alkyl), -COOH, -COO(C₁-C₄-Alkyl), -CONH(C₁-C₄-Alkyl), -CON(C₁-C₄-Alkyl) (C₁-C₂-alkyl), -SH, -CN, -NO₂, -SO(C₁-C₄-Alkyl), -SO₂(C₁-C₄-Alkyl), -SO₂NH(C₁-C₄-Alkyl) und -SO₂N(C₁-C₄-Alkyl) (C₁-C₂-alkyl), substituiert sein kann, darstellt;
-NR¹R² einen gesättigten 3 bis 8 gliedrigen carbocyclischen Ring bilden kann, der gegebenenfalls eine bis drei Doppelbindungen enthalten kann und worin ein oder zwei Ringkohlenstoffatome von solchen 5 bis 8 gliedrigen Ringen gegebenenfalls und unabhängig durch ein Sauerstoff- oder Schwefelatom oder durch NZ³, worin Z³ Wasserstoff, C₁-C₄-Alkyl, Benzyl oder C₁-C₄-Alkanoyl darstellt, ersetzt sind;
R³ Wasserstoff, C₁-C₄-Alkyl, -O(C₁-C₄-Alkyl), Chlor, Fluor, Brom, Jod, -CN, -S(C₁-C₄-Alkyl) oder -SO₂(C₁-C₄)-Alkyl darstellt, worin jede der (C₁-C₄-Alkyl)einheiten in den vorangehenden Gruppen R³ gegebenenfalls mit einem Substituenten R⁹, ausgewählt aus Hydroxy, Fluor und (C₁-C₂-Alkoxy), substituiert sein kann;
jedes R⁴ unabhängig Wasserstoff, (C₁-C₆-Alkyl), Fluor, Chlor, Brom, Jod, Hydroxy, Cyano, Amino, Nitro, -O(C₁-C₄-Alkyl), -N(C₁-C₄-Alkyl)(C₁-C₂-alkyl), -S(C₁-C₄-Alkyl), -SO(C₁-C₄-Alkyl), -SO₂(C₁-C₄-Alkyl), -CO(C₁-C₄-Alkyl), -C(=O)H oder -C(=O)O(C₁-C₄-Alkyl) darstellt, worin jede der (C₁-C₆-Alkyl)-und (C₁-C₄-Alkyl)einheiten in den vorangehenden Gruppen R⁴ gegebenenfalls eine oder zwei Doppel- oder Dreifachbindungen enthalten kann und gegebenenfalls mit einem oder zwei Substituenten, unabhängig ausgewählt aus Hydroxy, Amino, C₁-C₃-Alkoxy, Dimethylamino, Methylamino, Ethylamino, -NHC(=O)CH₃, Fluor, Chlor, C₁-C₃-Thioalkyl, -CN, -COOH, -C(=O)O(C₁-C₄-Alkyl), -C(=O)(C₁-C₄-Alkyl) und -NO₂, substituiert sein kann;
R⁵ Phenyl, Naphthyl, Thienyl, Benzothienyl, Pyridyl, Chinolyl, Pyrazinyl, Furanyl, Benzofuranyl, Benzothiazolyl, Benzisothiazolyl, Benzisoxazolyl, Benzimidazolyl, Indolyl, Benzoxazolyl oder C₃-C₈-Cycloalkyl darstellt, worin ein oder zwei der Kohlenstoffatome des Cycloalkylrings, die mindestens 5 Ringglieder enthalten, gegebenenfalls und unabhängig durch ein Sauerstoff- oder Schwefelatom oder durch NZ⁴, worin Z⁴ Wasserstoff, C₁-C₄-Alkyl oder Benzyl darstellt, ersetzt sind; und worin jede der vorangehenden Gruppen R⁵ mit einem bis vier Substituenten R¹² substituiert sein kann, worin ein bis drei der Substituenten unabhängig aus Chlor, C₁-C₆-Alkyl und -O(C₁-C₆-Alkyl) ausgewählt sein können und einer der Substituenten aus Brom, Jod, Formyl, -CN, -CF₃, -NO₂, -NH₂, -NH(C₁-C₄-Alkyl), -N(C₁-C₂-Alkyl)(C₁-C₆-alkyl), -C(=O)O(C₁-C₄-Alkyl), -C(=O)(C₁-C₄-Alkyl), -COOH, -SO₂NH(C₁-C₄-Alkyl), -SO₂N(C₁-C₂-Alkyl)(C₁-C₄-alkyl), -SO₂NH₂, -NHSO₂(C₁-C₄-Alkyl), -S(C₁-C₆-Alkyl) und -SO₂(C₁-C₆-Alkyl) ausgewählt sein kann und worin jede der C₁-C₄-Alkyl- und C₁-C₆-Alkyleinheiten der vorangehenden Gruppen R⁵ gegebenenfalls mit einem oder zwei Substituenten, unabhängig ausgewählt aus Fluor, Hydroxy, Amino, Methylamino, Dimethylamino und Acetyl, substituiert sein kann;
R⁷ Wasserstoff, C₁-C₄-Alkyl, Halogen, Cyano, Hydroxy, -O(C₁-C₄-Alkyl)-C(=O)(C₁-C₄-alkyl), -C(=O)O(C₁-C₄-Alkyl), -OCF₃, -CF₃, -CH₂OH, -CH₂O(C₁-C₄-Alkyl) darstellt;
R¹⁰ Wasserstoff, Hydroxy, Methoxy oder Fluor darstellt;
R¹¹ Wasserstoff oder C₁-C₄-Alkyl darstellt; und
Z NH, Sauerstoff, Schwefel, -N(C₁-C₄-Alkyl), -NC(=O)(C₁-C₂-Alkyl), -NC(=O)O(C₁-C₂-Alkyl) oder CR¹³R¹⁴ darstellt, worin R¹³ und R¹⁴ aus Wasserstoff, Trifluormethyl und Methyl unabhängig ausgewählt sind, mit der Ausnahme, dass einer von R¹³ und R¹⁴ Cyano sein kann;
mit der Maßgabe, dass: (a) es in den fünfgliedrigen Ringen der Struktur I keine zwei zueinander benachbarten Doppelbindungen geben darf; und (b) wenn R⁴ an Stickstoff gebunden ist, es kein Halogen, Cyano oder Nitro ist;
oder ein pharmazeutisch verträgliches Salz einer solchen Verbindung.

2. Verbindung nach Anspruch 1, worin: R¹ C₁-C₆-Alkyl, das gegebenenfalls mit einer Hydroxy-, Fluor-, CF₃- oder C₁-C₄-Alkylgruppe substituiert sein kann und gegebenenfalls eine Doppel- oder Dreifachbindung enthalten kann, darstellt und R² Benzyl, C₁-C₆-Alkyl, das gegebenenfalls eine Doppel- oder Dreifachbindung enthalten kann, darstellt, wobei die C₁-C₆-Alkyl- und die Phenyleinheit von dem Benzyl gegebenenfalls mit einer Fluor-, CF₃-, C₁-C₂-Alkyl-, C₁-C₂-Alkoxy- oder Chlorgruppe substituiert sein kann.

3. Verbindung nach Anspruch 1, worin: R³ Methyl, Ethyl, Chlor oder Methoxy darstellt; R⁴ Methyl, Ethyl oder Trifluormethyl darstellt; G Wasserstoff, Methyl, Ethyl darstellt oder E=G C=O, C=S darstellt; R⁵ Phenyl, Pyridyl, Pyrimidyl, das mit mehr als zwei Substituenten unabhängig ausgewählt aus C₁-C₄-Alkyl, -O(C₁-C₄-Alkyl), (C₁-C₄-Alkyl)-O-(C₁-C₄alkyl), CF₃, OCF₃, -CHO, (C₁-C₄-Alkyl)-OH, CN, Cl, F, Br, I und NO₂, substituiert ist, wobei jede der vorangehenden (C₁-C₄)-Alkylgruppen gegebenenfalls eine Doppel- oder Dreifachbindung enthalten kann, darstellt.

4. Verbindung nach Anspruch 1, worin A N, CH oder CCH₃ darstellt, die gegebenenfalls mit Fluor, Chlor, CF₃, C₁-C₄-Alkyl oder C₁-C₄-Alkoxy substituiert sein können.

5. Verbindung nach Anspruch 1, worin F NR⁴ darstellt.

6. Verbindung nach Anspruch 1, worin F CHR⁴ darstellt.

7. Verbindung nach Anspruch 1, worin F Stickstoff darstellt und doppelt an E gebunden ist.

8. Verbindung nach Anspruch 1, worin F Schwefel darstellt.

9. Verbindung nach Anspruch 1, worin E Kohlenstoff darstellt.

10. Verbindung nach Anspruch 1, worin E Stickstoff darstellt.

11. Verbindung nach Anspruch 1, worin E NR²⁵ darstellt und R²⁵ Wasserstoff, C₁-C₄-Alkyl oder -CF₃ darstellt.

12. Verbindung nach Anspruch 1, die ausgewählt ist aus:
2,5,6-Trimethyl-7-(1-propylbutyl)-4-(2,4,6-trimethylphenoxy)-7H-pyrrolo[2,3-d]pyrimidin;
1-(1-Ethylpropyl)-6-methyl-4-(2,4,6-trimethylphenylamino)-1,3-dihydroimidazo[4,5-c]pyridin-2-on;
9-(1-Ethylpropyl)-2-methyl-6-(2,4,6-trimethylphenylamino)-7,9-dihydropurin-8-on;
1-(1-Ethylpropyl)-6-methyl-4-(2,4,6-trimethylphenoxy)-1,3-dihydroimidazo[4,5-c]pyridin-2-on;
1-(1-Ethylpropyl)-6-methyl-4-(2,4,6-trimethylphenoxy)-1H-imidazo[4,5-c]pyridin;
1-(1-Ethylpropyl)-3,6-dimethyl-4-(2,4,6-trimethylphenoxy)-1,3-dihydroimidazo[4,5-c]pyridin-2-on; und
1-(1-Ethylpropyl)-3,6-dimethyl-4-(2,4,6-trimethylphenylamino)-1,3-dihydroimidazo[4,5-c]pyridin-2-on.

13. Pharmazeutische Zusammensetzung, umfassend eine Verbindung oder ein Salz davon nach einem vorangehenden Anspruch und ein pharmazeutisch verträgliches Verdünnungsmittel oder einen pharmazeutisch verträglichen Träger.

14. Verbindung oder Salz nach einem der Ansprüche 1 bis 12 oder eine Zusammensetzung davon nach Anspruch 13 zur Verwendung als ein Arzneimittel.

15. Verwendung einer Verbindung oder eines Salzes nach einem der Ansprüche 1 bis 12 oder einer Zusammensetzung nach Anspruch 13 zur Herstellung eines Arzneimittels zur Behandlung von (a) einer Störung, deren Behandlung durch Entgegenwirken von CRF bewirkt oder erleichtert werden kann, einschließlich, jedoch nicht begrenzt auf Störungen, die durch CRF induziert oder erleichtert werden, oder (b) einer Störung, ausgewählt aus entzündlichen Störungen, wie rheumatische Arthritis und Osteoartritis, Schmerz, Asthma, Psoriasis und Allergien; allgemeiner Angststörung; Panik; Phobien; obsessiv-compulsiver Störung; posttraumatischer Stressstörung; Hypertension; Tachycardie; Herzstauungsinsuffizienz; durch Stress induzierter Schlafstörungen; Schmerzwahrnehmung, wie Fibromyalgie; Stimmungsstörungen, wie Depression, einschließlich verherrschender Depression, Einzelepisodendepression, wiederkehrender Depression, durch Kindesentzug induzierter Depression und Postpartumdepression; Dysthemie; bipolare Störungen; Cyclothymie, Ermüdungssyndrom; Stress-induziertem Kopfschmerz; Krebs; reizbarem Darmsyndrom; Crohnscher Krankheit; spastischem Colon; Immunmangelvirusinfektionen beim Menschen; neurodegenerativen Erkrankungen, wie Alzheimer-Krankheit, Parkinson-Krankheit und Huntington-Krankheit; Gastrointestinaltraktströungen; Essstörungen, wie Anorexie und Bulimia nervosa; hämorrhagischem Stress; Stress-induzierten psychotischen Episoden; euthyroidem Krankheitssyndrom; Syndrom von ungeeignetem antidiarrhetischem Hormon; Fettsucht; Infertilität; Kopftrauma; Wirbelsäulentrauma; ischämischer neuronaler Schädigung; excitotoxischer neuronaler Schädigung; Epilepsie; Schlaganfall; Geschwüren; Immundysfunktionen, einschließlich Stress-induzierten Immundysfunktionen; Muskelspasmen; Harninkontinenz; seniler Demenz vom Alzheimer-Typ; Multiinfarktdemenz; amyotropher lateraler Sklerose; chemischen Abhängigkeiten und Süchten; Arzneimittel- und Alkoholentzugssymptomen; psychosozialem Zwergwuchs; und Hypoglycämie bei einem Säuger.

## Revendications

1. Composé de formule 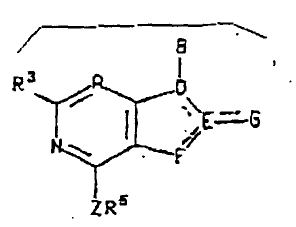 ou un de ses sels pharmaceutiquement acceptables, formule dans laquelle
les lignes discontinues représentent des doubles liaisons facultatives ;
A représente un atome d'azote ou un groupe CR⁷ ;
B représente un groupe -NR¹R², -CR¹R²R¹⁰, -C(=CR²R¹¹)R¹, -NHCR¹R²R¹⁰, -OCR¹R²R¹⁰, -SCR¹R²R¹⁰, -CR²R¹⁰NHR¹, -CR²R¹⁰OR¹, -CR²R¹⁰SR¹ ou -COR² ;
D représente un atome d'azote et est lié par une liaison simple à tous les atomes auxquels il est fixé ;
E représente un atome d'azote, un groupe CH ou un atome de carbone ;
F représente un atome d'oxygène ou de soufre, un groupe CHR⁴ ou NR⁴ lorsqu'il est lié par une liaison simple à E et F représente un atome d'azote ou un groupe CR⁴ lorsqu'il est lié par une double liaison à E ;
G, lorsqu'il est lié par une liaison simple à E, représente un atome d'hydrogène, un groupe alkyle en C₁ à C₄, -S(alkyle en C₁ à C₄), -O(alkyle en C₁ à C₄), NH₂, -NH(alkyle en C₁ à C₄) ou -N(alkyle en C₁ ou C₂)(alkyle en C₁ à C₄), dans lequel chacun des groupes alkyle en C₁ à C₄ de G peut être facultativement substitué avec un groupe hydroxy, -O(alkyle en C₁ ou C₂) ou fluoro ; G, lorsqu'il est lié par une double liaison à E, représente un atome d'oxygène ou de soufre ou un groupe NH ; et G, lorsque E représente un atome d'azote et est lié par une double liaison à D ou F, est absent ;
R¹ repréente un groupe alkyle en C₁ à C₆ facultativement substitué avec un ou deux substituants R⁸ choisis, indépendamment, entre des substituants hydroxy, fluoro, chloro, bromo, iodo, alkoxy en C₁ à C₄, CF₃, -C(=O)O(alkyle en C₁ à C₄), -OC(=O) (alkyle en C₁ à C₄), -OC(=O)N(alkyle en C₁ à C₄) (alkyle en C₁ ou C₂), -NHCO(alkyle en C₁ à C₄), -COOH, -COO(alkyle en C₁ à C₄), -CONH(alkyle en C₁ à C₄), -CON(alkyle en C₁ à C₄) (alkyle en C₁ ou C₂), -S(alkyle en C₁ à C₄), -CN, -NO₂, -SO(alkyle en C₁ à C₄), -SO₂(alkyle en C₁ à C₄), -SO₂NH(alkyle en C₁ à C₄) et -SO₂N(alkyle en C₁ à C₄) (alkyle en C₁ ou C₂), dans lesquels chacun des groupes alkyle en C₁ à C₄ dans les groupes R¹ précités peut contenir facultativement une ou deux doubles liaisons ou triples liaisons ;
R² représente un groupe alkyle en C₁ à C₁₂ Qui peut contenir facultativement une à trois doubles ou triples liaisons, aryle ou (alkylène en C₁ à C₄)aryle, dans lequel ledit groupe aryle et le groupement aryle dudit groupe (alkylène en C₁ à C₄)aryle est choisi entre des: groupes phényle, naphtyle, thiényle, benzothiényle, pyridyle, quinolyle, pyrazinyle, pyrimidinyle, imidazolyle, furannyle, benzofurannyle, benzothiazolyle, isothiazolyle, pyrazolyle, pyrrolyle, indolyle, pyrrolopyridyle, oxazolyle et benzoxazolyle ; cycloalkyle en C₃ à C₈ ou (alkylène en C₁ à C₆)(cycloalkyle en C₃ à C₈), dans lequel un ou deux des atomes de carbone dudit groupe cycloalkyle et des groupements cycloalkyle penta- à octogonaux dudit groupe (alkylène en C₁ à C₆) (cycloalkyle en C₃ à C₈) peuvent être facultativement et, indépendamment, remplacés par un atome d'oxygène ou de soufre ou par un groupe NZ² dans lequel Z² est choisi entre un atome d'hydrogène, des groupes alkyle en C₁ à C₄, benzyle et alcanoyle en C₁ à C₄, et chacun des groupes R² précités peut être facultativement substitué avec un à trois substituants choisis, indépendamment, entre des substituants chloro, fluoro, hydroxy et alkyle en C₁ à C₄ ou avec un substituant choisi entre des substituants bromo, iodo, alkoxy en C₁ à C₆, -OC(=O)(alkyle en C₁ à C₆), -OC(=O)N(alkyle en C₁ à C₄)(alkyle en C₁ ou C₂), -S(alkyle en C₁ à C₆), amino, -NH(alkyle en C₁ ou C₂), -N(alkyle en C₁ ou C₂)(alkyle en C₁ à C₄), -N(alkyle en C₁ à C₄)-CO-(alkyle en C₁ à C₄), -NHCO(alkyle en C₁ à C₄), -COOH, -COO(alkyle en C₁ à C₄), -CONH(alkyle en C₁ à C₄), -CON(alkyle en C₁ à C₄)-(alkyle en C₁ ou C₂), -SH, -CN, -NO₂, -SO(alkyle en C₁ à C₄), -SO₂(alkyle en C₁ à C₄), -SO₂NH(alkyle en C₁ à C₄) et -SO₂N(alkyle en C₁ à C₄)(alkyle en C₁ ou C₂) ;
le groupe -NR¹R² peut former un noyau carbocyclique tri- à octogonal qui peut contenir facultativement une à trois doubles liaisons, dans lequel un ou deux des atomes de carbone du noyau de ces noyaux penta- à octogonaux peuvent facultativement et, indépendamment, être remplacés par un atome d'oxygène ou de soufre ou par un groupe NZ³ dans lequel Z³ représente un atome d'hydrogène, un groupe alkyle en C₁ à C₄, benzyle ou alcanoyle en C₁ à C₄ ;
R³ représente un atome d'hydrogène, un groupe alkyle en C₁ à C₄, -O(alkyle en C₁ à C₄), chloro, fluoro, bromo, iodo, -CN, -S(alkyle en C₁ à C₄) ou -SO₂(alkyle en C₁ à C₄) dans lequel chacun des groupements alkyle en C₁ à C₄ dans les groupes R³ précités peut être facultativement substitué avec un substituant R⁹ choisi entre des groupes hydroxy, fluoro et alkoxy en C₁ ou C₂ ;
chaque groupe R⁴ représente, indépendamment, un atome d'hydrogène, un groupe alkyle en C₁ à C₆, fluoro, chloro, bromo, iodo, hydroxy, cyano, amino, nitro, -O(alkyle en C₁ à C₄), -N(alkyle en C₁ à C₄)(alkyle en C₁ ou C₂), -S(alkyle en C₁ à C₄), -SO(alkyle en C₁ à C₄), -SO₂(alkyle en C₁ à C₄), -CO(alkyle en C₁ à C₄), -C(=O)H ou -C(=O)O(alkyle en C₁ à C₄), dans lequel chacun des groupements alkyle en C₁ à C₆ et alkyle en C₁ à C₄ dans les groupes R⁴ précités peut contenir facultativement une ou deux doubles liaisons ou triples liaisons et peut être facultativement substitués avec un ou deux substituants choisis, indépendamment, entre des substituants hydroxy, amino, alkoxy en C₁ à C₃, diméthyl-amino, méthylamino, éthylamino, -NHC(=O)CH₃, fluoro, chloro, thioalkyle en C₁ à C₃, -CN, -COOH, -C(=O)O(alkyle en C₁ à C₄), -C(=O)(alkyle en C₁ à C₄) et -NO₂ ;
R⁵ représente un groupe phényle, naphtyle, thiényle, benzothiényle, pyridyle, quinolyle, pyrazinyle, furannyle, benzofurannyle, benzothiazolyle, isothiazolyle, benzisothiazolyle, benzisoxazolyle, benzimidazolyle, indolyle, benzoxazolyle ou cycloalkyle en C₃ à C₈, dans lequel un ou deux des atomes de carbone desdits noyaux cycloalkyle qui contiennent au moins 5 membres dans le noyau peuvent être facultativement et, indépendamment, remplacés par un atome d'oxygène ou de soufre ou par un groupe NZ⁴ dans lequel Z⁴ représente un atome d'hydrogène, un groupe alkyle en C₁ à C₄ ou benzyle ; et chacun des groupes R⁵ précités est substitué avec un à quatre substituants R¹², un à trois desdits substituants pouvant être choisis, indépendamment, entre des substituants chloro, alkyle en C₁ à C₆ et -O(alkyle en C₁ à C₆) et un desdits substituants pouvant être choisis entre des substituants bromo, iodo, formyle, -CN, -CF₃, -NO₂, -NH₂, -NH(alkyle en C₁ à C₄), -N(alkyle en C₁ ou C₂)(alkyle en C₁ à C₄), -O(=O)O(alkyle en C₁ à C₄), -C(=O)(alkyle en C₁ à C₄), -COOH, -SO₂NH(alkyle en C₁ à C₄), -SO₂N(alkyle en C₁ ou C₂)(alkyle en C₁ à C₄), -SO₂NH₂, -NHSO₂(alkyle en C₁ à C₄), -S(alkyle en C₁ à C₆) et -SO₂(alkyle en C₁ à C₆) et chacun des groupements alkyle en C₁ à C₄ et alkyle en C₁ à C₆ dans les groupes R⁵ précités pouvant être facultativement substitué avec un ou deux substituants choisis, indépendamment, entre des substituants fluoro, hydroxy, amino, méthylamino, diméthylamino et acétyle ;
R⁷ représente un atome d'hydrogène, un groupe alkyle en C₁ à C₄, halogéno, cyano, hydroxy, -O(alkyle en C₁ à C₄), -C(=O)(alkyle en C₁ à C₄), -C(=O)O(alkyle en C₁ à C₄), -OCF₃, -CF₃, -CH₂OH ou -CH₂O(alkyle en C₁ à C₄) ;
R¹⁰ représente un atome d'hydrogène, un groupe hydroxy, méthoxy ou fluoro ;
R¹¹ représente un atome d'hydrogène ou un groupe alkyle en C₁ à C₄ ; et
Z représente un groupe NH, un atome d'oxygène, un atome de soufre, un groupe -N(alkyle en C₁ à C₄), -NC(=O)(alkyle en C₁ ou C₂), NC(=O)O(alkyle en C₁ ou C₂) ou CR¹³R¹⁴ dans lequel R¹³ et R¹⁴ sont choisis, indépendamment, entre un atome d'hydrogène, des groupes trifluorométhyle et méthyle, sauf qu'un des groupes R¹³ et R¹⁴ peut représenter un groupe cyano ;
sous réserve que : (a) dans les noyaux pentagonaux de structure I, il ne peut exister deux doubles liaisons adjacentes l'une à l'autre ; et (b) lorsque R⁴ est fixé à un atome d'azote, il ne représente pas un groupe halogéno, cyano ou nitro ;
ou un sel pharmaceutiquement acceptable d'un tel composé.

2. Composé suivant la revendication 1, dans lequel : R¹ représente un groupe alkyle en C₁ à C₆, qui peut être facultativement substitué avec un groupe hydroxy, fluoro, CF₃ ou alkoxy en C₁ à C₄ et qui peut contenir facultativement une double ou triple liaison i; et R² représente un groupe benzyle, alkyle en C₁ à C₆ qui peut contenir facultativement une double ou triple liaison, ledit groupe alkyle en C₁ à C₆ et le groupement phényle dudit groupe benzyle pouvant être facultativement substitués avec un groupe fluoro, CF₃, alkyle en C₁ ou C₂, alkoxy en C₁ ou C₂ ou chloro.

3. Composé suivant la revendication 1, dans lequel : R³ représente un groupe méthyle, éthyle, chloro ou méthoxy ; R⁴ représente un groupe méthyle, éthyle ou trifluorométhyle ; G représente un atome d'hydrogène, un groupe méthyle, éthyle ou bien E=G représente un groupe C=O, C=S ; R⁵ représente un groupe phényle, pyridyle, pyrimidyle qui est substitué avec plus de deux substituants choisis, indépendamment, entre des substituants alkyle en C₁ à C₄, -O(alkyle en C₁ à C₄), (alkyle en C₁ à C₄)-O-(alkyle en C₁ à C₄), CF₃, OCF₃, -CHO, (alkyle en C₁ à C₄)-OH, CN, Cl, F, Br, I et NO₂, chacun des groupes alkyle en C₁ à C₄ précités pouvant contenir facultativement une double ou triple liaison.

4. Composé suivant la revendication 1, dans lequel A représente N, un groupe CH ou CCH₃ qui peut être facultativement substitué avec un substituant fluoro, chloro, CF₃, alkyle en C₁ à C₄ ou alkoxy en C₁ à C₄.

5. Composé suivant la revendication 1, dans lequel F représente un groupe NR⁴.

6. Composé suivant la revendication 1, dans lequel F représente un groupe CHR⁴.

7. Composé suivant la revendication 1, dans lequel F représente un atome d'azote et est lié par une double liaison à E.

8. Composé suivant la revendication 1, dans lequel F représente un atome de soufre.

9. Composé suivant la revendication 1, dans lequel E représente un atome de carbone.

10. Composé suivant la revendication 1, dans lequel E représente un atome d'azote.

11. Composé suivant la revendication 1, dans lequel E représente un groupe NR²⁵ et R²⁵ représente un atome d'hydrogène, un groupe alkyle en C₁ à C₄ ou -CF₃.

12. Composé suivant la revendication 1, qui est choisi entre les suivants :
2, 5, 6-triméthyl-7- (1-propylbutyl)-4-(2,4,6-triméthylphénoxy)-7H-pyrrolo[2,3-d]pyrimidine ;
1- (1-éthylpropyl)-6-méthyl-4-(2,4,6-triméthylphénylamino)-1,3-dihydro-imidazo[4,5-c]pyridine-2-one ;
9-(1-éthylpropyl)-2-méthyl-6-(2,4,6-triméthylphénylamino)-7,9-dihydropurine-8-one ;
1-(1-éthylpropyl)-6-méthyl-4-(2,4,6-triméthylphénoxy)-1,3-dihydro-imidazo[4,5-c]pyridine-2-one ;
1-(1-éthylpropyl)-6-méthyl-4-(2,4,6-triméthylphénoxy)-lH-imidazo[4,5-c]pyridine ;
1- (1-éthylpropyl)-3,6-diméthyl-4-(2,4,6-triméthylphénoxy)-1,3-dihydro-imidazo[4,5-c]pyridine-2-one ; et
1-(1-éthylpropyl)-3,6-diméthyl-4-(2,4,6-triméthylphénylamino)-1,3-dihydro-imidazo[4,5-c]pyridine-2-one.

13. Composition pharmaceutique comprenant un composé ou un de ses sels suivant l'une quelconque des revendications précédentes, et un diluant ou support pharmaceutiquement acceptable.

14. Composé ou sel suivant l'une quelconque des revendications 1 à 12 ou composition le contenant suivant la revendication 13, destiné à être utilisé comme médicament.

15. Utilisation d'un composé ou sel suivant l'une quelconque des revendications 1 à 12, ou d'une composition suivant la revendication 13, pour la production d'un médicament destiné au traitement (a) d'une affection dont le traitement peut être effectué ou facilité par antagonisation du CRF, comprenant, mais à titre nullement limitatif, des affections induites ou facilitées par le CRF, ou (b) d'une affection choisie entre des troubles inflammatoires tels que l'arthrite rhumatoïde et l'ostéoarthrite, la douleur, l'asthme, le psoriasis et des allergies ; le syndrome d'anxiété généralisée ; la panique ; des phobies ; le syndrome obsessionnel compulsif ; le syndrome de stress post-traumatique ; l'hypertension ; la tachycardie ; l'insuffisance cardiaque congestive ; des troubles du sommeil induit par un stress ; une perception douloureuse telle que la fibromyalgie ; des troubles de l'humeur tels que la dépression, comprenant la dépression majeure, la dépression à épisode unique, la dépression récidivante, la dépression induite par des sévices sur enfants et la dépression du postpartum ; la dysthymie ; des troubles bipolaires ; la cyclothymie ; le syndrome de fatigue ; les céphalées induites par un stress ; le cancer ; le syndrome du côlon irritable ; la maladie de Crohn ; le côlon spastique ; des infections par le virus de l'immunodéficience humaine ; des maladies neurodégénératives telles que la maladie d'Alzheimer, la maladie de Parkinson et la maladie de Huntington ; des troubles gastro-intestinaux ; des troubles de l'alimentation tels que l'anorexie mentale et la boulimie mentale ; un stress hémorragique ; des épisodes psychotiques induits par un stress ; le syndrome de maladie euthyroïdienne ; le syndrome d'hormone antidiarrhéique inappropriée ; l'obésité ; la stérilité ; des traumatismes crâniens ; un traumatisme de la moelle épinière ; une altération neuronale ischémique ; une altération neuronale excitotoxique ; l'épilepsie ; un ictus ; des ulcères ; des dysfonctionnements immunitaires comprenant des dysfonctionnements immunitaires induits par un stress ; les spasmes musculaires ; l'incontinence urinaire ; la démence sénile de type Alzheimer ; la démence artériopathique ; la sclérose latérale amyotrophique ; des dépendances et accoutumances chimiques ; des symptômes de sevrage de médicaments et de l'alcool ; le nanisme psychosocial et l'hypoglycémie chez un mammifère.
